(19)

Europäisches
Patentamt
European
Patent Office
Office européen
des brevets

(11) **EP 1 754 725 A2**

(12) **EUROPEAN PATENT APPLICATION**

(43) Date of publication:
    **21.02.2007 Bulletin 2007/08**

(51) Int Cl.:
    *C08F 20/00* *(2006.01)*

(21) Application number: **06017061.0**

(22) Date of filing: **16.08.2006**

(84) Designated Contracting States:
    **AT BE BG CH CY CZ DE DK EE ES FI FR GB GR
HU IE IS IT LI LT LU LV MC NL PL PT RO SE SI
SK TR**
    Designated Extension States:
    **AL BA HR MK YU**

(30) Priority: **17.08.2005 JP 2005236878**

(71) Applicant: **Nippon Shokubai Co.,Ltd.
Osaka-shi, Osaka 541-0043 (JP)**

(72) Inventors:
  • **Irie, Yoshio
    Himeji-shi
    679-2101 Hyogo (JP)**

• **Saswabe, Masazumi
  Kakogawa-shi
  675-0122 Hyogo (JP)**
• **Kato, Seiji
  Himeji-shi
  671-1242 (JP)**
• **Dairoku, Yorimichi
  Himeji-shi
  672-8085 Hyogo (JP)**

(74) Representative: **Graf von Stosch, Andreas et al
Bosch Graf von Stosch Jehle
Patentanwaltsgesellschaft mbH
Flüggenstrasse 13
80639 München (DE)**

(54) **Production method of water-absorbent resin, water-absorbent resin, and usage of water-absorbent resin**

(57)    The subject invention produces a water-absorbent resin by carrying out stationary polymerization of an ethylenically-unsaturated monomer containing acrylic acid and/or acrylate; cutting the resulting hydrous gelatinous polymer; and drying the hydrous gelatinous polymer which mainly contains angular particles having 6 smooth planes. The resulting water-absorbent resin particles are not aggregate, and can be efficiently dried. By coating at least a part of the surface of each of the particles with an adhesion block agent before, after, and/or at the time of cutting process, the adhesion preventing effect further increases.

**EP 1 754 725 A2**

**Description**

FIELD OF THE INVENTION

The present invention relates to a production method of water-absorbent resin which prevents adhesion between hydrous gelatinous polymers and enables the polymers to be efficiently dried. The present invention also relates to the water-absorbent resin, and usage of the water-absorbent resin.

BACKGROUND OF THE INVENTION

It is a well-known fact that polymerization of an aqueous solution of ethylenically-unsaturated monomer produces hydrous gelatinous polymer which has a water-absorbent property (disclosed in Document 1, for example). The hydrous gelatinous polymer is an elastic half-solid gel, which is cut into pieces and dried to be a water-absorbent resin product serving as a water-absorbent agent.

However, in the cutting process, the hydrous gelatinous polymers are adhered to each other unless some kind of special treatment is done. The adhesion causes, for example, some difficulties in continuously cutting the polymers with a cutting device, thus decreasing work efficiency. To prevent such adhesion, surfactant, polymer multivalent alcohol or the like has been used (Documents 2 and 3).

Meanwhile, unreacted monomers in the hydrous gelatinous polymer (residual monomer, hereinafter) act as impurities in the polymer, which may cause odor or other sanitary problems. Therefore, the amount of residual monomers is preferably maximally reduced. Conventional methods for reducing the residual monomer include a usage of a larger amount of radical polymerization initiator and divisional supply of radical polymerization initiator (Documents 4 to 10).

Further, Document 11 discloses a method of spraying multivalent metal salt aqueous solution to the dry powder hydrophilic polymer particles 20 mass % or lower in moisture content. This method has an effect of increasing the absorption of the water-absorbent material.

However, this conventional method cannot ensure sufficient prevention of adhesion between the hydrous gelatinous polymer particles.

Further, even though the specific causes are unknown, the methods according to Documents 6 and 7 have a problem of foreign odor which occurs after the subsequent heating process. The odor is assumed to be caused by a component deriving from a mixture of the added reducing material, the impurities generated from the materials and the like. Further, in the method of Document 8, even though the amount of residual monomer is securely reduced, the residual monomer amount of the absorbent greatly varies. Consequently, in some cases, the amount of residual monomer was not sufficiently reduced, or the method produces some B-grade products.

Further, in the residual monomer reduction methods according to Documents 4 and 5, the obtained water-absorbent resin becomes colored in some cases, and the value of product decreases. Further, in these methods, reduction of residual monomer requires various severe reaction conditions, and therefore the physicality may decrease due to degradation of polymer. Therefore, the desired degree of reduction of residual monomer and maintenance/improvement of absorption property, such as absorbencies under pressure and under no pressure, may not be simultaneously ensured.

Document 4 teaches the use of polyethylene glycol and a surfactant as an adhesion block agent, but does not have specific description of the treatment of the hydrous gelatinous polymer with these agents. Moreover, in the method of Document 5 using surfactant, polymer multivalent alcohol etc., the particle size distribution of the hydrous gelatinous polymer is large, and the shapes of the hydrous gelatinous polymer particles are not even. The particles are therefore likely to agglutinate.

Further, the method of Document 11 in which a multivalent metal salt aqueous solution is splayed into the dry polymer particles has been publicly-known, but the original purpose of this method is not to prevent adhesion between the hydrous gelatinous polymer particles. There has been no disclosure of a technology for securely preventing adhesion between the hydrous gelatinous polymer particles by coating the particles with a multivalent metal salt aqueous solution or the like.

[Document 1]

Japanese Laid-Open Patent Application Tokukaihei 08-92307 (published on April 9, 1996)

[Document 2]

Japanese Laid-Open Patent Application Tokukaisho 61-110510 (published on May 28, 1986)

[Document 3]

Japanese Laid-Open Patent Application Tokukaihei 11-349687 (published on December 21, 1999)

[Document 4]

US Patent No. 4973632

[Document 5]

US Patent No. 5998553

[Document 6]
Japanese Laid-Open Patent Application Tokukosho 63-7203 (published on February 16, 1988)
[Document 7]
US Patent No. 2004-0068057
[Document 8]
Japanese Laid-Open Patent Application Tokukohei 07-98847 (published on October 25, 1995)
[Document 9]
Japanese Laid-Open Patent Application Tokukaihei 04-106108 (published on April 8, 1992)
[Document 10]
US Patent No. 5229488
[Document 11]
Japanese Laid-Open Patent Application Tokukohei 05-40780 (published on June 21, 1993)

SUMMARY OF THE INVENTION

The present invention is made in view of the foregoing conventional problems, and an object is to provide a production method of a water-absorbent resin, and the water-absorbent resin produced through the method, which ensure prevention of adhesion of hydrous gel particles and therefore facilitate drying of the particles.

The present invention also suggests usage of the water-absorbent resin. The present invention more preferably relates to a production method of a water-absorbent resin, and the water-absorbent resin produced through the method, which ensure reduction in residual monomer amount.

The inventors of the present invention had an intensive study to solve the foregoing problems, and created angular hydrous polymer particles each of which has six smooth plains. At least a part of the surface of each particle is coated with an adhesion block agent containing at least one kind of compounds classified into multivalent metal salt, multivalent alcohol, or surfactant at least partially by a specific method. With these particles, the cutting process can be more easily performed, and because they do not aggregate, they can be efficiently dried. Further, the present invention also achieves reduction in residual monomer amount by drying hydrous gelatinous polymer specified in mass average particle diameter. The inventors thus completed the present invention.

A production method of water-absorbent resin according to the present invention comprises the steps of:

(1) carrying out stationary polymerization of a sheet-type ethylenically-unsaturated monomer containing acrylic acid and/or acrylate at a ratio of 30 mole% to 100 mole% (excluding a crosslinking agent) so as to produce a hydrous gelatinous polymer containing moisture at a ratio of 40 mass % to 70 mass %;

(2a) cutting the hydrous gelatinous polymer so as to produce hydrous gelatinous polymer particles, the hydrous gelatinous polymer particles containing particles 3mm to 10mm in mass average particle diameter, each of which has 4 - 12 planes, the hydrous gelatinous polymer particles containing said particles in an amount of 50 mass % to 100 mass % with respect to the mass of the hydrous gelatinous polymer: or

(2b) drying the hydrous gelatinous polymer until its moisture content becomes 5 mass % to 60 mass % and then cutting the hydrous gelatinous polymer so as to produce hydrous gelatinous polymer particles, the hydrous gelatinous polymer particles containing particles 2mm to 10mm in mass average particle diameter, each of which has 4 - 12 planes, the hydrous gelatinous polymer particles containing said particles in an amount of 50 mass % to 100 mass % with respect to the mass of the hydrous gelatinous polymer;

(3) coating at least a part of the surface of each of the hydrous gelatinous polymer particles with an adhesion block agent containing at least one kind of compounds classified into multivalent metallic salt, multivalent alcohol or a surfactant, the coating being performed before, after, and/or at the time of cutting the hydrous gelatinous polymer; and

(4) drying the hydrous gelatinous polymer particles having been coated by adhesion block agent.

With this method, the hydrous gel polymer contains particles with 4 - 12 smooth planes in an amount of 50 mass % to 100 mass %, and each of the particles is coated with an adhesion block agent, which serves as a lubricant.

Therefore, at the time of cutting the hydrous gelatinous polymer into particles or after the particles are made by the cutting, the particles do not aggregate. The particles can also be efficiently dried.

Further, since the moisture content of the hydrous gelatinous polymer is 40 mass % to 70 mass %, and the mass average particle diameter is large, namely 3mm to 10mm, the residual monomer can be sufficiently reacted in the drying process. Therefore, the resulting water-absorbent resin has a less amount of residual monomer, and immune to odor or other sanitary defects.

A cutting device for cutting a sheet-type hydrous gelatinous polymer according to the present invention comprises: at least one vertical-cutting blade for vertically cutting the hydrous gelatinous polymer; at least one horizontal-cutting spinning blade and at least one fixed blade for vertically cutting the hydrous gelatinous polymer; and at least one spraying means for spraying and/or diffusing an adhesion block agent onto the vertical-cutting blade, the horizontal-cutting spinning blade, the fixed blade and/or the hydrous gelatinous polymer.

With this arrangement, the adhesion block agent can be efficiently applied by spraying/ diffusion to the angular particles of the hydrous gelatinous polymer produced by vertical/horizontal cutting. Therefore, it is possible to reduce adhesion between the particles. The method is therefore useful for production of particles uniform in shape and are not aggregate, or for production of water-absorbent resin causing a less amount of residual monomer.

A water-absorbent resin according to the present invention is produced through the steps of:

(1) carrying out stationary polymerization of an ethylenically-unsaturated monomer containing acrylic acid and/or acrylate at a ratio of 30 mole% to 100 mole% so as to produce a hydrous gelatinous polymer;
(2) cutting the hydrous gelatinous polymer so as to produce hydrous gelatinous polymer particles containing particles each of which has 4 - 12 planes, the hydrous gelatinous polymer particles containing said particles in an amount of 50 mass % to 100 mass % with respect to the mass of the hydrous gelatinous polymer; and
(3) drying the hydrous gelatinous polymer particles, the water-absorbent resin being 2mm to 10mm in mass average particle diameter, and 0 to 0.25 in logarithm standard deviation of particle size distribution.

The water-absorbent resin is obtained by drying the hydrous gel polymer particles uniform in particle size. The hydrous gel polymer particles are therefore sufficiently prevented from adhering to each other in the manufacturing process. The water-absorbent resin is also sufficiently dried.

A screener for screening the dried water-absorbent resin according to the present invention comprises a gas spraying mechanism.

This screener allows removal of excessively-large particles and excessively-small particles from the dried water-absorbent resin. Therefore, it is possible to adjust the mass average particle diameter of the water-absorbent resin to a desired value. With the water-absorbent resin uniform in particle size, the display effect of the water-absorbent resin increases.

A plant-raising water retaining agent according to the present invention contains the water-absorbent resin produced through the foregoing method, or the water-absorbent resin according to the present invention specified above. With the less amount of residual monomer which decreases the physicality of the resin, the water-absorbent resin of the present invention is immune to odor or other sanitary defects. The plant-raising water retaining agent of the present invention is therefore capable of capturing a sufficient amount of water. As long as it contains a certain amount of water, the water retaining agent constantly supplies water to plant. On this account, the plant is not required to be frequently watered. The water-retaining agent is also superior in display effect.

An artificial ice for display according to the present invention contains the water-absorbent resin produced through the foregoing method, or the water-absorbent resin according to the present invention specified above. With the less amount of residual monomer which decreases the physicality of the resin, the water-absorbent resin of the present invention is immune to odor or other sanitary defects. An artificial ice for display according to the present invention is capable of capturing a sufficient amount of water. Therefore, as long as it contains a certain amount of water, the appearance close to a real ice cube can be kept for a long time. The artificial ice for display according to the present invention is therefore superior in display effect.

Additional objects, features, and strengths of the present invention will be made clear by the description below. Further, the advantages of the present invention will be evident from the following explanation in reference to the drawings.

BRIEF DESCRIPTION OF THE DRAWINGS

Figure 1 is a vertical cross-sectional view showing a structure of a defoaming device.
Figure 2 is a vertical cross-sectional view showing a structure of a deaeration film module.
Figure 3 is a vertical cross-sectional view showing a structure of a cutting device according to one embodiment of the present invention.
Figure 4 is a vertical cross-sectional view showing a coated scraper.
Figure 5 is an explanatory view showing an upper view of vertical-cutting blades 1 and 2.
Figure 6 is an explanatory view showing a portion where the engagement between vertical-cutting blades (roll cutter) 1 and 2 is established.
Figure 7 is a magnified view of the two blades of Figure 6 engaged with each other.
Figure 8 is a lateral view of a structure of a screening device according to one embodiment of the present invention.
Figure 9 is a cross-sectional view showing a structure of the first screening section shown in Figure 8.
Figure 10 is a schematic projection view showing a structure of the second screening section of Figure 8.

Figure 11 is a schematic view of a device for measuring the dust amount of the water-absorbent resin.
Figure 12 is a schematic projection view showing a structure of the second screening section of Figure 8.
Figure 13 is a cross-sectional view showing a structure of a vibration planar screener according to one embodiment of the present invention.
Figure 14 is a schematic projection view showing a structure of the second screening section of Figure 13.
Figure 15 is a schematic view of a device for measuring dust amount of the water-absorbent resin.

DESCRIPTION OF THE EMBODIMENTS

The embodiment of the present invention is described below. The present invention is however not limited to the embodiment. First, the following explains a production method of water-absorbent resin according to the present invention.

(1. Production method of water-absorbent resin)

According to one of the embodiments of the present invention, a production method of water-absorbent resin comprising the steps of:

(1) carrying out stationary polymerization of a sheet ethylenically-unsaturated monomer containing acrylic acid and/or acrylate at a ratio of 30 mole% to 100 mole% (excluding the crosslinking agent) so as to produce a hydrous gelatinous polymer whose moisture content is 40 mass % to 70 mass %;
(2) cutting the hydrous gelatinous polymer so that the hydrous gelatinous polymer contains, at a ratio of 50 mass % to 100 mass %, particles 3 to 10mm in mass average particle diameter having 4-12 planes, or cutting the hydrous gelatinous polymer after drying the hydrous gelatinous polymer until its moisture content becomes 5 mass % to 60 mass % so that the hydrous gelatinous polymer contains particles with 4-12 planes at a ratio of 50 mass % to 100 mass % with respect to the gross mass % of the hydrous gelatinous polymer;
(3) coating at least a part of the surface of the hydrous gelatinous polymer with an adhesion block agent containing at least one kind of compound classified into multivalent metallic salt, multivalent alcohol or a surfactant before, after, and/or at the time of cutting the hydrous gelatinous polymer; and
(4) drying the hydrous gelatinous polymer having been coated by adhesion block agent.

The hydrous gelatinous polymer is produced by polymerizing ethylenically-unsaturated monomer preferably in the presence of a small amount of crosslinking agent, and coating at least a part of the surface of the hydrous gelatinous polymer with an adhesion block agent containing at least one kind of compound of multivalent metallic salt, multivalent alcohol or a surfactant. The coating is performed before, after, and/or at the time of cutting the hydrous gelatinous polymer. Note that, in this specification, "hydrous gelatinous polymer" includes (i) a hydrous gelatinous polymer in which the polymers are not crosslinked, and (ii) a crosslinked hydrous gelatinous polymer in which the polymers are crosslinked. Further, "mass" and "mass %" in the present specification are equivalent to "weight" and "weight %".
The ethylenically-unsaturated monomer used as the material of the hydrous gelatinous polymer is water soluble. Examples of the ethylenically-unsaturated monomer include monomer having an acid group, such as (meth) acrylic acid, β-acrylyl oxy propionic acid, maleic acid (or maleic anhydride), fumaric acid, crotonic acid, itaconic acid, cinnamic acid, 2-(meth) acrylylethane sulfonic acid, 2-(meth) acrylylpropane sulfonic acid, 2-(meth) acrylamide -2-methylpropane sulfonic acid, vinyl sulfonic acid, styrene sulfonic acid, allylsulfonate, vinyl phosphonic acid, 2-(meth) acryloyloxyethyl phosphoric acid, or (meth) acryloxyalkane sulfonic acid; and its alkaline metal salts or alkaline earth metal salt, ammonium salts, alkylamine salt; dialkylaminoalkyl (meth) acrylate, such as N,N-dimethylaminoethyl (meth)acrylate, N,N-dimethylaminopropyl (meth)acrylate, N,N-dimethylaminopropyl (meth)acrylamide, and quaternary salt thereof (product obtained from reaction with alkyl hydride, product obtained from reaction with dialkylsulfate or the like); dialkylaminohydroxyalkyl (meth) acrylate and quaternary salt thereof; N- alkylvinyl pyridinium halide; hydroxyalkyl (meth) acrylate such as hydroxymethyl (meth) acrylate, 2-hydroxyethyl (meth) acrylate, or 2-hydroxypropyl (meth) acrylate; acrylamide, methacrylamide, N-ethyl(meth)acrylamide, N-n-propyl(meth)acrylamide, N-isopropyl(meth)acrylamide, or N,N-dimethyl(meth) acrylamide; alcoxypolyethyleneglycol (meth)acrylate, such as methoxypolyethyleneglycol (meth)acrylate, polyethyleneglycol mono (meth)acrylate; vinyl pyridine, N-vinyl pyridine, N-vinyl pyrrolidone, N-acrylylpiperidine; N-vinylacetamide. One or more kinds of ethylenically-unsaturated monomer are used as required.
Any of the foregoing ethylenically-unsaturated monomers may be used but the monomer preferably contains acrylic acid and/or acrylate at a ratio of 30 mole% to 100 mole%, more preferably 50 mole% to 100 mole%, further preferably 90 mole% to 100 mole%. If the content is less than 30 mole%, the resulting hydrous gelatinous may have poor absorbency and security, and the polymerization reactivity may also decrease. Further, the versatility and economical efficiency may also decrease. Here, the acrylate means alkali metallic salt of acrylic acid, alkali earth metallic salt of

5

acrylic acid, ammonium salt of acrylic acid, hydroxyammonium salt of acrylic acid, amine salt of acrylic acid, alkylamine salt of acrylic acid.

Among the examples of water soluble salts, the sodium salt or potassium salt are particularly preferable.

One or plural kinds of these acrylic acid salt monomers may be used as required. The average molecular amount (degree of polymerization) of water-absorbent resin is not particularly limited.

To produce the hydrous gelatinous polymer, the monomer composition containing an ethylenically-unsaturated monomer as a main component is polymerized preferably in the presence of a small amount of crosslinking agent. The monomer composition may contain other kind of monomer having a copolymerization property with respect to the ethylenically-unsaturated monomer (copolymerization monomer), however, the amount of the monomer is limited to a level not decreasing the hydrophilicity of the resulting ethylenically-unsaturated monomer.

Examples of the copolymerization monomer include a (meth) acrylic acid ester, such as methyl (meth) acrylate, ethyl (meth) acrylate, or butyl (meth) acrylate; or a hydrophobic monomer, such as vinyl acetate, or vinyl propionate. One or plural kinds of these copolymerization monomers may be used as required.

The crosslinking agent used in the polymerization of the monomer component may be a compound having a plurality of vinyl groups in a molecule; or a compound having in a molecule a plurality of functional groups reacting with a carboxyl group or a sulfuric group. One or plural kinds of these crosslinking agents may be used as required.

Examples of the compound having a plurality of vinyl groups in a molecule include N,N-dimethylenebis (meth) acrylamide, (poly) ethyleneglycol di(meth) acrylate, (poly)propyleneglycol di(meth)acrylate, trimethylolpropanetri(meth) acrylate, trimethylolpropanedi(meth) acrylate, glyceroltri(meth)acrylate, glycerolacrylatemethacrylate, ethyleneoxide denatured trimethylolpropanetri(meth) acrylate, pentaerythritoltetra(meth)acrylate, dipentaerythritolhexa(meth)acrylate, N, N-diallylacrylamide, triallylcyanurate, triallylisocyanurate, triallylphosphate, triallyl amine, diallyloxy acetate bis(N-vinylcarboxylic amide) and tetraallyloxyethane.

Examples of the compound having in a molecule a plurality of functional groups reacting with a carboxyl group or a sulfuric group includes a multivalent alcoholic compound, such as a (poly) ethyleneglycol, diethyleneglycol, propyleneglycol, triethyleneglycol, tetraethyleneglycol, 1,3-propanediol, dipropyleneglycol, 2, 2, 4-trimethyl-1, 3-pentanediol, polypropyleneglycol, (poly) glycerin, 2-butane-1, 4-diol, 1, 4-butanediol, 1,5-pentanediol, 1, 6-hexandiol, 1, 2-cyclohexandimethanol, 1, 2-cyclohexanol, trimethylol propane. diethanolamine, triethanolamine, polyoxy propylene, oxyethyleneoxypropylene block copolymerizate, pentaerythritol, or sorbitol; an epoxy compound such as (poly) ethyleneglycol diglycidyl ether, (poly) glycerol polyglycidyl ether, diglycerol polyglycidyl ether, (poly) propyleneglycol diglycidyl ether, or glycidol; a multivalent amine compound such as ethylenediamine, diethylenetriamine, triethylenetetramine, tetraethylenepentamine, pentaethylenehexamine, polyamidepolyamine, polyethylene-imine; a condensate of the multivalent amine or a haloepoxy compound; a multivalent isocyanate compound such as 2,4-trilene diisocyanate or hexamethylene diisocyanate; a multivalent oxazoline such as 1, 2-ethylenebis oxazoline; a silane coupling agent such as γ-glycidoxypropyltrimethoxysilane or γ-aminopropyltriethoxysilane; an alkylene carbonate compound such as 1,3-dioxolan-2-one, 4-methyl-1,3-dioxolane-2-one; 4,5-dimethyl-1,3-dioxolane-2-one; 4,4-dimethyl-1,3-dioxolane-2-one; 4-ethyl-1,3-dioxolane-2-one; 4-hydroxymethyl-1,3-dioxolane-2-one; 1,3-dioxane-2-one, 4-methyl-1,3-dioxane-2-one, 4,6-dimethyl-1,3-dioxane-2-one, 1, 3-dioxopane-2-one; a haloepoxy compound such as epichlorohydrin; and hydroxide or chloride compound of zinc, calcium, magnesium, alminium, iron, or zirconium.

The amount of the crosslinking agent is not particularly limited, but preferably 0.0001 mole% to 10 mole%, more preferably 0.001mole% to 1 mole%, with respect to the monomer component. In the present invention, the polymerization of the monomer component is carried out by aqueous solution polymerization, specifically stationary polymerization on a tray or belt, or polymerization in a kneader. Among these, stationary polymerization on a moving belt is preferable, as it enables the resulting polymer to be cut in a continuous manner.

The thickness of the polymer sheet is not particularly limited but stationary polymerization is preferably performed so that the resulting sheet becomes 3mm to 10mm in thickness. In other words, the sheet-shaped hydrous gelatinous polymer produced through the polymerization preferably has a thickness of 3mm to 10mm. By specifying the thickness in the stationary polymerization, it is possible to effectively reduce the residual monomer in the drying process.

More specifically, when the sheet of hydrous gelatinous polymer is cut vertically and horizontally, in the most of the cases, the resulting gel has an angular shape with 6 smooth planes, and this means that vertically and horizontally cutting the sheet of hydrous gelatinous polymer creates a gel 3mm to 10mm in mass average diameter having an angular shape with 6 smooth planes. As described later, a hydrous gelatinous polymer 3mm to 10mm in mass average diameter is useful for reducing the residual monomer content in the water-absorbent resin having been through a drying process. Therefore, the stationary polymerization is preferably performed so that the resulting sheet becomes 3mm to 10mm in thickness.

Further, when the ethylenically-unsaturated monomer is subjected to aqueous solution polymerization, the polymerization may be performed in a continuous manner or a batch manner. The process may be performed under any of: atmospheric pressure state, depressiruzation state, and compression state. Note that, the polymerization reaction may be performed in the air, but the polymerization reaction under air current of inactive gas such as nitrogen, helium, argon, carbon dioxide is more preferable.

In the beginning of the polymerization reaction, a polymerization initiator or an active energy such as X-ray, electron ray, ultraviolet, or electromagnetic ray may be used, for example.

The polymerization initiator is not particularly limited, and a thermal decomposition initiator or a photodecomposition initiator may be used. The amount is preferably in a range of 0.001 to 5 mal %, more preferably 0.01 to 0.5 mol %. Examples of the thermal decomposition initiators include persulfate such as sodium persulfate, potassium persulfate, and ammonium persulfate; peroxide such as hydrogen peroxide, t-butylperoxide, and methylethylketoneperoxide; and an azo compound such as azonitryl compound, azoamidine compound, circular azoamidine compound, azoamide compound, alkylazo compound, 2,2'-azobis (2-amidinopropane) dihydrochloride, or 2,2'-azobis [2-(2-imidazoline-2-yl) propane] dihydrochloride).

Examples of the photodecomposition initiator include benzoin derivative, benzyl derivative, acetophenone derivative, benzophenone derivative, and azo compound. One kind or two or more kinds are selected from these initiators. Further, it may be so arranged that: also a reducer for promoting decomposition of each polymerization initiator is used so that a combination of the polymerization initiator and the reducer serves as a redox initiator. Examples of the above reducer include (bi)sulfurous acid (salts) and L-ascorbic acid.

The amount of dissolved oxygen in the ethylenically-unsaturated monomer aqueous solution is preferably constant to ensure stable start of the polymerization reaction. To further facilitate the polymerization, amount of dissolved oxygen in the ethylenically-unsaturated monomer aqueous solution is preferably reduced. To be more specific, the amount of dissolved oxygen is preferably not more than 8mg/l, more preferably not more than 6mg/l, further preferably not more than 2mg/1.

When the polymerization reaction is started with air bubbles in the aqueous solution which is generated by deaeration, the bubbles remain in the sheet, and the display effect of the water-absorbent resin decreases. To prevent this, both deaeration and defoaming are required.

The deaeration and defoaming of the residual oxygen from the ethylenically-unsaturated monomer aqueous solution may be performed by blowing of inactive gas, vacuum degassing, membrane degassing, addition of reducing agent or the like. The following describes these methods.

(a. Blowing of inactive gas)

The blowing of inactive gas may be performed by (I) blowing inactive gas into a tube containing ethylenically-unsaturated monomer aqueous solution which is connected to the polymerization device, or (II) by blowing inactive gas into a tank containing the ethylenically-unsaturated monomer. In either way, it is necessary to remove the bubbles of supplied inactive gas and bubbles of generated oxygen before the polymerization is started.

In the method (I) in which inactive gas is blown into a tube containing ethylenically-unsaturated monomer aqueous solution which is connected to the polymerization device, the defoaming may be performed by separating the bubbles and the liquid using spiral flow, or by pouring the ethylenically-unsaturated monomer aqueous solution into a deaeration tank and leaving the solution as such until the bubbles are removed. The first method using spiral flow may be performed with a "QUICK TORON®" (product of Nippon Oil Corporation), for example.

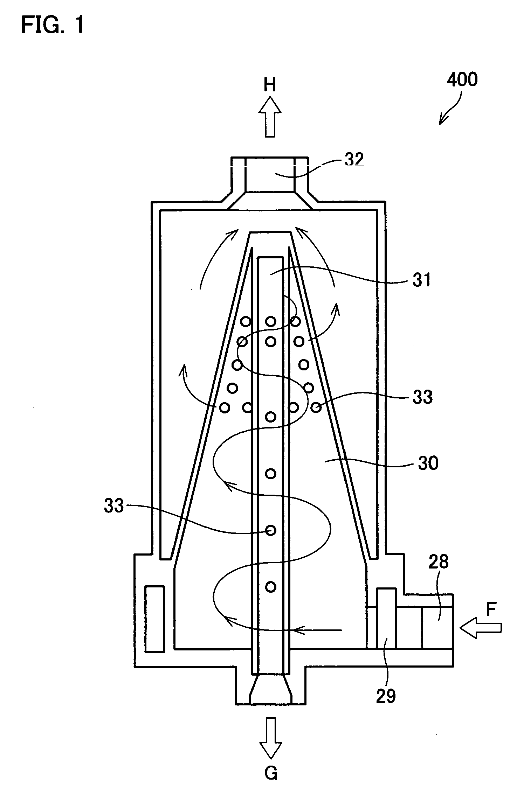

A defoaming device such as the "QUICK TORON®" is explained below with reference to Figure 1. A defoaming device 400 is a cylinder-shaped device, and contains a cyclone room 30 having a circular-cone shape. In the middle of the cyclone room 30, a bubble removing tube 31 is provided. Further, an inlet 28 for supplying a liquid containing bubbles, and a auxiliary spiral flow room 29 are provided. Above the defoaming device 400, an outlet hole 32 is provided.

The fluid supplied toward the arrow F is then supplied into the cyclone room 30. After the removal of bubbles by the cyclone room 30, the fluid passes through a large number of small holes before discharged from the outlet 32 toward the arrow H.

Meanwhile, the bubbles with a small amount of liquid are gathered to the center of the cyclone room 30, and then are discharged toward the arrow G via the bubble removing tube 31.

In the method (II) in which inactive gas is blown into a tank containing the ethylenically-unsaturated monomer, the solution having been supplied with inactive gas may be placed still until the bubbles disappear.

(b. Vacuum deaeration)

In this method, the amount of dissolved oxygen is reduced by reducing pressure of the ethylenically-unsaturated monomer aqueous solution. This method may be performed by reducing pressure of the ethylenically-unsaturated monomer aqueous solution, but may otherwise be performed by using a deaeration pump, such as a deaeration pump ASP-0310/ASP-0510 (product of *Yokota Seisakusho*).

(c. Membrane degassing)

This is a kind of vacuum deaeration in which the outside of the deaeration membrane module is depressurized when the ethylenically-unsaturated monomer aqueous solution passes through inside the deaeration membrane module. As a result, the dissolved oxygen in the ethylenically-unsaturated monomer aqueous solution is discharged to the outside of the deaeration membrane module.

The deaeration membrane may be a porous membrane or a separation membrane. For example, the separation membrane may be a silicon resin non-porous membrane NAGASEP M40-A, M40-B, M-60-B, or M80-B provided by *NAGAYANAGI* CO. LTD. Another example may be a deaeration module in which a non-porous membrane is sandwiched between polyethylene porous membranes; namely MHF0504MBFT, MHF1704, MHF3504, MHF304EED, MGF304KMD, or MHF0498P provided by MITSUBISHI RAYON ENGINEERING CO., LTD.

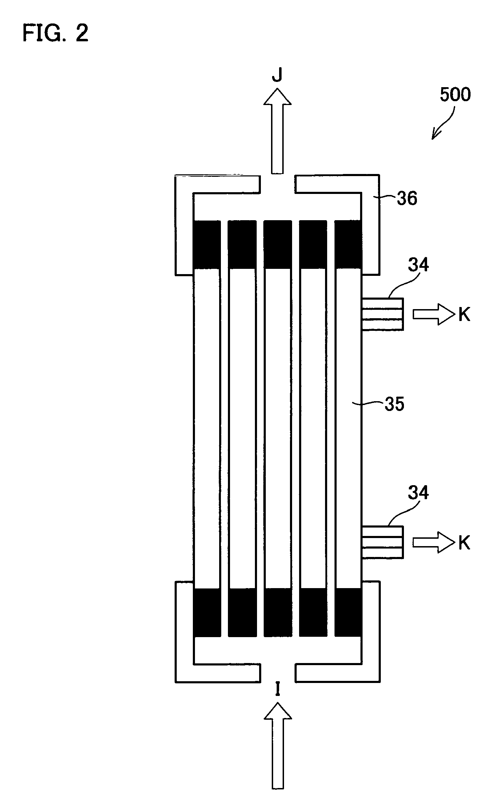

Figure 2 shows a schematic view of a deaeration membrane module. The deaeration membrane module 500 has a plurality of deaeration membranes 35 inside of a cover 36. The deaeration membrane module 500 also has vacuum pumps each of which extends from the lateral side of the deaeration membrane 35 through the tubes 34. With this arrangement, the pressure can be reduced in the direction denoted by the arrow K. First, fluid containing dissolved gas such as dissolved oxygen is supplied to the deaeration membrane module 500 in the direction of the arrow I. After that, the dissolved gas passes through the deaeration membrane 35 in the direction of the arrow K. As a result, the deaerated fluid is discharged toward the arrow J.

(d. Addition of reducing agent)

This method carries out chemical deaeration by adding hydrodin, sodium sulfite, or sodium bisulfite to the ethylenically-unsaturated monomer aqueous solution.

With these methods, or individually, ultrasonic deaeration/defoaming may be performed.

The bubbles generated in the gel sheet during the polymerization reaction are not desired as they decrease the display effect of the water-absorbent resin. To prevent this defect, it is preferable to (I) carry out deaeration or defoaming of the ethylenically-unsaturated monomer aqueous solution; (II) suppress heat generation during the polymerization, for example, by setting the temperature of the ethylenically-unsaturated monomer aqueous solution to be at or less than 30°C, more preferably at or less than 25°C, further preferably at or less than 20°C; or (III) start polymerization by polymerization reaction using ultra violet, which is induced by a photodecomposition initiator. Note that, the maximum temperature of the hydrous gelatinous polymer in the polymerization is preferably 60°C to 100°C, more preferably 70°C to 90°C, further preferably 80°C to 90°C. The excessively high/low temperature causes the following defects. If the temperature is greater than 100°C, the ethylenically-unsaturated monomer aqueous solution will suddenly boil. On the other hand, if the temperature is less than 80°C, the productivity decreases.

The gross light quantity of ultra violet used for the polymerization reaction is preferably $500mJ/cm^2$ to $5000mJ/cm^2$, more preferably $600mJ/cm^2$ to $4000mJ/cm^2$.

If the gross light quantity is less than $500mJ/cm^2$, a larger amount of photodecomposition initiator is required.

This is not desirable. On the other hand, if the gross light quantity is more than $5000mJ/cm^2$, the polymerization reaction suddenly occurs, and the heat suddenly generated may make the ethylenically-unsaturated monomer aqueous solution immediately boil. Further, the gel sheet is irradiated with a larger amount of energy than necessary, which causes degradation/decomposition of the hydrous gelatinous polymer.

The UV lamp for generating ultra violet may be a black light mercury lamp, a metal halide lamp, or a high-pressure mercury lamp. One or plural of these lamps may be used according to need. The most preferred is using both a metal halide lamp and a black light mercury lamp, by which the gross light quantity can be adjusted.

In the case of polymerizing the monomer component in the presence of a crosslinking agent, water is preferably used as a solvent. More specifically, the monomer compound and the crosslinking agent are preferably dissolved in water. With this arrangement, the resulting water-absorbent resin has a superior water-absorbent property.

The concentration of the monomer component in the aqueous solution (hereinafter referred to as a monomer aqueous solution) preferably falls within 30 mass % to 60 mass %. When the concentration of monomer component falls below 30 mass %, the ratio of water soluble component in the resulting water-absorbent resin increases. Meanwhile, when the concentration of monomer component exceeds 60 mass %, there may be a difficulty in controlling the reaction temperature. With the above method and arrangement, a hydrous gelatinous polymer with a moisture content of 40 mass % to 70 mass % is produced.

Further, the solvent of the monomer aqueous solution may be made by mixing water and a water-soluble organic solvent. Examples of the organic solvent include methyl alcohol, ethyl alcohol, acetone, dimethylsulfoxide, ethyleneglycolmonomethylether, glycerin, (poly)ethyleneglycol, (poly) propyleneglycol and alkylenecarbonate. One or more kind of these organic solvents may be used according to the circumstance.

In the cutting process, the sheet-shaped hydrous gelatinous polymer is cut vertically and horizontally so that the hydrous gelatinous polymer contains, at a ratio of 50 mass % to 100 mass %, angular-shaped particles each of which has 4-12 smooth planes; more preferably, so that the resulting hydrous gelatinous polymer contains, at a ratio of 50 mass % to 100 mass %, further preferably 80 mass % to 100 mass %, still further preferably 90 mass % to 100 mass %, particularly preferably 95 mass % to 100 mass %, angular-shaped particles each of which has 6 smooth planes.

If the content of angular-shaped particles with 6 smooth planes in the resulting hydrous gelatinous polymer is less than 50 mass %, the unevenness of shape may cause a decrease in display performance when the hydrous gelatinous polymer is dried to be a water-absorbent resin and is swollen by absorption of aqueous solution.

Vertically and horizontally cutting the sheet of hydrous gelatinous polymer creates a gel is mainly constituted of particles each having an angular shape with 6 smooth planes. However, because of errors in the cutting process, the polymer also contains some polyhedron particles. More specifically, the resulting hydrous gelatinous polymer contains, at a ratio of 50 mass % to 100 mass %, angular-shaped particles each of which has 4 - 12 smooth planes.

Hydrous gelatinous polymer particles with less than 4 planes or with more than 12 planes are not preferable, as they may cause a decrease in display performance when the hydrous gelatinous polymer is dried to be a water-absorbent resin and is swollen by absorption of aqueous solution.

The cutting process may be performed by any cutting means such as a roller-type cutter, guillotine cutter, slicer, roll-cutter, shredder, or scissors. One or plural kinds of cutting means may be arbitrarily selected.

The following explains an adhesion block agent.

The adhesion block agent contains at least one kind of compound classified into multivalent metallic salt, multivalent alcohol or a surfactant before, after, and/or at the time of cutting the hydrous gelatinous polymer. The adhesion block agent covers at least a part of the surface of the hydrous gelatinous polymer so as to prevent the hydrous gelatinous polymer particles from adhering to each other.

The amount of the adhesion block agent is not particularly limited, but preferably 0.0015 mass % to 35 mass %, more preferably 0.002 mass % to 30 mass %, further preferably 0.002 mass % to 25 mass %. If the amount is less than 0.0015 mass %, the particulate hydrous gelatinous polymer obtained through the cutting process has some difficulties in releasing from the blade, and tends to easily aggregate again. On the other hand, If the amount is more than 35 mass %, the effect is not proportional to the increase of amount, and also may decrease the physicality of the final product of water-absorbent resin.

Examples of the multivalent metal include aluminum chloride, polychlorinated aluminum, aluminum sulfate, aluminum nitrate, bis potassium aluminum sulfate, bis sodium aluminum sulfate, potassium alum, ammonium alum, sodium aluminate, calcium chloride, calcium nitrate, magnesium chloride, magnesium sulfate, magnesium nitrate, zinc chloride, zinc nitrate, iron chloride (III), cerium chloride (III), ruthenium chloride (III), yttrium chloride (III), chromium chloride (III), zirconium sulfate, hexafluoro potassium zirconium, hexafluoro sodium zirconium, zirconium ammonium carbonate, zirconium potassium carbonate, sodium zirconium carbonate, zirconium acetate, and propionic acid zirconium.

Among these, salts having crystal water of these substances are more preferable because of the superior dissolving property to water or aqueous solutions. An aluminum compound is particularly preferable, and among them, aluminum chloride, polychlorinated aluminum, aluminum nitrate, bis potassium aluminum sulfate, bis sodium aluminum sulfate, potassium alum, ammonium alum, sodium alum, sodium aluminate are preferable. Aluminum sulfate is particularly preferable. Examples of typically preferred aluminum sulfate include crystal hydrate of aluminum sulfate 18-hydrate, aluminum sulfate 14-18 hydrate or the like. One or plural kinds of these substances may be used according to circumstances.

The multivalent metal salt is preferably dissolved in water. The concentration of the multivalent metal salt aqueous solution is not particularly limited but preferably 1 mass % to 50 mass %, more preferably 3 mass % to 40 mass %, further preferably 10 mass % to 20 mass %.

If the concentration of the multivalent metal salt aqueous solution is less than 1 mass %, a large amount of multivalent metal salt aqueous solution is required to ensure the blade-releasing property. This results in an unwanted great increase of energy required for the drying process. On the other hand, if the concentration of the multivalent metal salt aqueous solution is more than 50 mass %, the blade-releasing property increases, but the water-dissolving property decreases, raising some difficulties in evenly spraying the solution onto the surface of the hydrous gelatinous polymer. In addition to this inadequacy, the increase in usage amount is not economically desirable.

Examples of the multivalent alcohol include ethyleneglycol, polyethyleneglycol, diethyleneglycol, triethyleneglycol, tetraethyleneglycol, 1,3-propanediol, dipropyleneglycol, polypropyleneglycol, 2,2,4-trimethyl-1,3-pentanediol, glycerin, 2-butene-1,4-diol, 1,3-butanediol, 1,4-butandiol, 1,5-pentanediol, 1,6-hexanediol, 1,2-cyclohexanedimethanol, 1,2-cyclohexanol, trimethylolpropane, and 2,3,4-trimethyl-1,3-pentandiol. Among these, propyleneglycol, is particularly preferable because of its versatility. One or plural kinds of these substances may be used according to circumstances.

Examples of the surfactant include anionic surfactant, nonionic surfactant, cationic surfactant, and zwitter-ionic surfactant. Examples of anionic surfactant include fatty acid salt such as mixed fatty acid sodium soap, partially-hydrogenated tallowate sodium soap, stearic acid sodium soap, oleic acid potassium soap, or ricinus potassium soap; alkylsulfate ester salt such as sodium lauryl sulfate, higher alcohol sodium sulfate, or lauryl sulfate triethanolamine; alkylbenzene sulfonic acid such as docecyl benzene sodium sulfonic acid; alkyl naphthalene sulfonic acid salt such as alkyl naphthalene sodium sulfonic acid; alkylsulfo succinate such as dialkylsulfo sodium succinate; alkyldiphenylether disulfonic acid such as alkyldiphenylether sodium disulfonic acid; alkyl phosphoric salt such as potassium alkyl phosphoric salt; polyoxyethylene alkyl (alkyl allyl) ester salt sulfate such as polyoxyethylene lauryl sodium ether, polyoxyethylene alkyl ether sodium sulfate, polyoxyethylene alkyl ether triethanol amine sulfate, or polyoxyethylene alkyl phenyl ether sodium sulfate; special reaction type anionic surfactant; special carbonic acid surfactant; naphthalene sulfonic acid formalin condensate such as sodium salt of β-naphthalene sulfonic acid formalin condensate, or sodium salt of special aromatic sulfonic acid formalin condensate; special polycarbonic acid type polymer surfactant; and polyoxyethylene alkyl phosphoric acid ester.

Examples of the nonionic surfactant include polyolefin oxide such as polyethyleneglycol, polypropyleneglycol, polyethyleneglycol-polypropyleneglycol block copolymer; polyoxyethylene alkyl ether such as polyoxyethylene lauryl ether, polyoxyethylene cetyl ether, polyoxyethylene stearyl ether, polyoxyethylene oleyl ether, polyoxyethylene higher alcohol ether; polyoxyethylene alkyl aryl ether such as polyoxyethylene nonylphenyl ether; polyoxyethylene derivative, sorbitan fatty acid ester such as sorbitan monolaurate, sorbitan palmitate, sorbitan monostearate, sorbitan tristearate, sorbitan sesquioleate, sorbitan distearate; polyoxyethylene sorbitan fatty acid ester such as polyoxyethylene sorbitan monolaurate, polyoxyethylene sorbitan palmitate, polyoxyethylene sorbitan monostearate, polyoxyethylene sorbitan tristearate, polyoxyethylene sorbitan monooleate, or polyoxyethylene sorbitan trioleate; polyoxyethylene sorbitol fatty acid ester such as tetraoleicacid polyoxyethylene sorbit; glycerin fatty acid ester such as glycerol monostearate, glycerol monooleate, or self-emulsifiable glycerol monostearate; polyoxyethylene fatty acid ester such as polyethyleneglycol monolaurate, polyethyleneglycol monostearate, polyethyleneglycol distearate, or polyethyleneglycol monooleate; polyoxyethylene alkyl amine; polyoxyethylene hard ricinus; and alkyl alkanol amide.

Examples of cationic surfactant and ampho-ion surfactant include alkyl amone salt such as coconut amine acetate, or stearyl amine acetate; quaternary ammonium salt such as lauryl trimethyl ammonium chloride, stearyl trimethyl ammonium chloride, cetyl trimethyl ammonium chloride, distearyl dimethyl ammonium chloride, alkyl benzyl dimethyl ammonium chloride, alkyl betaine such as lauryl betaine, stearyl betaine, laurylcarboxymethylhydroxyethyl imidazolium betaine; and amine oxide such as lauryldimethyl amine oxide. In addition to the surfactants listed above, fluorinated surfactant, siloxane surfactant etc. may be used.

Among these, polypropyleneglycol and polyethyleneglycol-polypropyleneglycol block copolymer are particularly preferable. With these surfactants, the amount to be added to the hydrous gelatinous polymer can be reduced. Moreover, these surfactants do not inhibit the physicality (eg. absorbency under pressure) of the hydrous gelatinous polymer when the hydrous gelatinous polymer is subjected to surface treatment after the surfactant is added thereto. These surfactant are highly secure in handling.

The adhesion block agent contains at least one of the compounds classified into multivalent metal salt, multivalent alcohol, or surfactant. When plural kinds of those are used as an adhesion block agent, those compounds classified into either multivalent metal salt, multivalent alcohol, or surfactant may be mixed, or may be used separately. In the case of mixing the multivalent metal salt, multivalent alcohol, and surfactant, their amounts are not limited, but a preferable combination is multivalent metal salt and multivalent alcohol, in which case the amount is preferably 20 mass % or less with respect to the amount of the adhesion block agent.

The coating with the adhesion block agent is required at least for a part of the surface of the hydrous gelatinous polymer, but it is more preferable to coat whole surface to ensure the blade-releasing property. The "coating" does not necessarily mean percolation of the adhesion block agent into the hydrous gelatinous polymer, but the adhesion block agent may percolate into the hydrous gelatinous polymer to an extent not damaging the function of the hydrous gelatinous polymer.

The moisture content of the hydrous gelatinous polymer is not particularly limited but preferably 40 mass % to 70 mass %, more preferably 45 mass % to 65 mass %, further preferably 50 mass % to 60 mass %.

If the moisture content is less than 40 mass %, the temperature of heat generated by the polymerization reaction increases, and induces many bubbles in the hydrous gelatinous polymer. This is not preferable. ON the other hand, a moisture content of more than 70 mass % is not preferable either, as it may decrease efficiency in the polymerization reaction. Further the residual monomer can be sufficiently reacted, but too much moisture remains even after the drying process, and therefore more energy is required for the drying. As a result, the drying process becomes less efficient.

Further, the mass average particle diameter of the hydrous gelatinous polymer is preferably in a range of 3mm to 10mm, more preferably 3mm to 8mm. The logarithm standard deviation of the particle diameter distribution of the hydrous gelatinous polymer is preferably not less than 0 and not more than 0.25.

In a general hydrous gelatinous polymer, the amount of residual monomer tends to decrease as the particle diameter increases provided that the drying is carried out under a constant condition. More specifically, in the drying process of the hydrous gelatinous polymer, the surface is first dried (moisture is evaporated due to increase in temperature), and the inner portion is then gradually dried. In the case of hydrous gelatinous polymer of small particles, the moisture in the particles deep inside of the polymer becomes dried relatively quickly. This slows down reaction of the polymerization initiator remaining in the residual monomer and the hydrous gelatinous polymer. Because of this, the amount of residual monomer tends to increase. A polymer having a mass average particle diameter = less than 3mm is not preferable because such a small particle diameter tends to increase the amount of residual monomer.

On the other hand, if the hydrous gelatinous polymer has a large particle diameter, the evaporation of moisture inside the gel does not occur immediately after the increases of the inner temperature of the polymer gel in the drying process. As a result, the residual monomer reacts with the polymerization initiator, and the amount of the residual monomer tends to decrease. If the mass average particle diameter of the hydrous gelatinous polymer is more than 10mm, the amount of the residual monomer tends to decrease for the same reason, but a longer time (energy) is required to dry the inner part of the gel. This decreases the efficiency of work, and is therefore not preferable.

If the mass average particle diameter of the hydrous gelatinous polymer falls within a range of 3mm to 10mm, and the logarithm standard deviation of the particle diameter distribution of the hydrous gelatinous polymer is not less than 0 and not more than 0.25, the particle size of the hydrous gelatinous polymer is relatively large and the distribution is even. Therefore, the residual monomer more easily reacts with the polymerization initiator in the drying process.

The "mass average particle diameter" designates an average particle size of the particles filtered by a standard sieve. Further, the logarithm standard deviation designates distribution of the average particle diameter. The methods of calculating the average particle diameter and the logarithm standard deviation are explained in "Example" below.

The adhesion block agent can be sprayed or diffused by any publicly-known spraying means, for example, an air-mixing type spraying device such as an atomizer, 1-fluid nozzle or 2-fluid nozzle; a liquid injecting pump; a brush; or a roller. The type and number of the spraying means of the present invention are not particularly limited.

The method of spraying the adhesion block agent is not particularly limited as long as it ensures that effect of blocking adhesion of the hydrous gelatinous polymer which is given by the adhesion block agent. The method however preferably includes a step of splaying and/or diffusing the adhesion block agent onto a vertical-cutting blade, a horizontal-cutting spinning blade, a fixed blade and/or the hydrous gelatinous polymer. In other words, as long as it ensures the foregoing effect, the adhesion block agent may be directly sprayed/diffused onto the hydrous gelatinous polymer, or may be sprayed/ diffused onto the vertical-cutting blade, the horizontal-cutting spinning blade and/or the fixed blade, so that the adhesion block agent is applied to the hydrous gelatinous polymer from the blades.

Further, by performing a mixture process as the coating process in addition to the cutting process, it becomes possible to add the adhesion block agent by spraying it into the resin particles. The mixture device is not limited as long as it has a general liquid/solid mixing function. Lödige mixer or Nauta mixer are however preferable.

The amount of the adhesion block agent applied to the vertical-cutting blade, the horizontal-cutting spinning blade, the fixed blade and/or the hydrous gelatinous polymer is not particularly limited. The amount is however preferably decided so that the content of the adhesion block agent in the hydrous gelatinous polymer becomes in a range of 0.0015 mass % to 35 mass %, more preferably 0.0015 mass % to 25 mass %, further preferably 0.0015 mass % to 20 mass %, most preferably 0.0015 mass % to 18 mass %, with respect to the solid content of the water-absorbent resin.

If the spraying/diffusing amount is less than 0.0015 mass %, the hydrous gelatinous polymer is not sufficiently coated with the adhesion block agent, that is, the adhesion blocking effect is not fully ensured. On the other hand, if the spraying/ diffusing amount is more than 35 mass %, greater energy is required for drying the gel, and also the amount of dust increases. Further, when multivalent metal salt is used, the crosslinking reaction on the gel surface is facilitated, and the absorbency decreases. Besides, depending on the type of multivalent metal, erosion of the cutting device may become more intense.

The method of including a chelate agent and/or an ultraviolet absorbent may be spraying and/or diffusion. The mixture device is not limited as long as it has a general liquid mixing function. Lödige mixer or Nauta mixer are however preferable.

Further, among the foregoing adhesion block agents, the surfactant or the multivalent alcohol may be included in the aqueous solution of the ethylenically-unsaturated monomer or in the hydrous gelatinous polymer. The method of including the adhesion block agent in the hydrous gelatinous polymer is not particularly limited. For example, the inclusion can be done by adding a surfactant and/or multivalent alcohol into an aqueous solution of the ethylenically-unsaturated monomer containing 30-100 mole% acrylic acid and/or acrylate, and evenly mixing them before polymerizing them.

By containing at least one kind of the compounds classified into surfactant or multivalent alcohol into the hydrous gelatinous polymer, it is possible to reduce adhesion between the surfaces of the hydrous gelatinous polymers, and/or the cross-sectional planes of the hydrous gelatinous polymers after the cutting process.

The amount of the surfactant and/or the multivalent alcohol is not particularly limited, but is preferably in a range of 0.001 to 10 mass %, more preferably 0.01 to 8 mass %, further preferably 0.03 to 5 mass %. When the amount of the surfactant and/or the multivalent alcohol is less than 0.001 mass %, the effect of reducing the adhesion between the cross-sectional surfaces decreases, and the particles of hydrous gelatinous polymer tend to aggregate again. On the other hand, when the amount of the surfactant and/or the multivalent alcohol is more than 10 mass %, the transparency of the water-absorbent resin decreases, which results in, for example, white turbidity, which decreases the display effect.

Further, a chelate agent and/or an ultraviolet absorbent may be applied on the surface (incl. cross-section) of the hydrous gelatinous polymer or may be included in the hydrous gelatinous polymer. The method of applying/ including the chelate agent and/or the ultraviolet absorbent into the hydrous gelatinous polymer is not particularly limited. For example, the chelate agent and/or an ultraviolet absorbent can be sprayed onto the hydrous gelatinous polymer by any publicly-known spraying means, such as an air-mixing type spraying device including an atomizer, 1-fluid nozzle or 2-fluid nozzle; or a liquid injecting pump.

Further, as with the case of adhesion block agent, the chelate agent and/or the ultraviolet absorbent may be sprayed and/or diffused onto the fixed blade and/or the spinning blade, which respectively vertically and horizontally cut the hydrous gelatinous polymer. As a result, the chelate agent and/or the ultraviolet absorbent are adhered to the hydrous gelatinous polymer so that they are applied to the surfaces (incl. cross-section) of the hydrous gelatinous polymer or included in the hydrous gelatinous polymer.

The chelate agent is a publicly-known compound having a metal-ion-sealing function, but otherwise not particularly limited. Examples of chelate agent include (1) aminocarbonic acid and salts thereof, (2) monoalkylamide citrate and monoalkenylamide citrate and their salts, (3) moronic acid monoalkylamide and moronic acid monoalkenylamide and their salts, (4) monoalkyl phosphate ester and monoalkenyl phosphate ester and their salts, (5) N-acylate glutamic acid and N-acylate asparaginic acid and their salts, (6) β-diketone derivative, and (7) tropolone derivative. One or plural kinds of chelate agent is used according to the circumstances.

(1) In terms of the metal-ion-sealing function, the aminocarbonic acid or the salt preferably has three or more carboxylic groups. Examples of such an aminocarbonic acid or the salt include nitrilotriacetic, ethylenediaminetetraacetic, diethylenetriaminepentaacetic, triethylenetetraaminehexaacetic, cyclohexane-1,2-diaminetetraacetic, N-hydroxyethylethylenediamintoriacetic, ethyleneglycoldiethyletherdiaminetetraacetic, ethylenediaminetetra propionic acid, N-alkyl-N'-carboxymethylasparaginic acid, N-alkenyl-N'-carboxymethylasparaginic acid, and their alkali metal salts, alkali earth metal salts, ammonium salts, and amine salts.

(2) The monoalkylamide citrate and monoalkenylamide citrate and their salts may be obtained by dehydrated condensation of alcohol and citric acid, for example.

(3) The moronic acid monoalkylamide and moronic acid monoalkenylamide and their salts may be obtained by adding α-olefin to moronic acid, and hydrolyzing the mixture.

(4) Examples of the monoalkyl phosphate ester and monoalkenyl phosphate ester and their salts include lauryl phosphate, stearyl phosphate.

(5) Examples of N-acylate glutamic acid and N-acylate asparaginic acid and their salts include *"amisoft* HS-11" or "GS-11" (products of Ajinomoto corporation).

(6) Examples of β-diketone derivative include acetylacetone and benzoilacetone.

(7) Examples of tropolone derivative include tropolone, β-thujaplicin, β-thujaplicin etc.

In terms of light resistance of chelate agent, the aminocarbonic acid or the salt preferably has three or more carboxylic groups. Further, diethylenetriaminepentaacetic, triethylenetetraaminehexaacetic, cyclohexane-1,2-diaminetetraacetic and N-hydroxyethylethylenediamintoriacetic and the salt are particularly preferable.

The ultraviolet absorbent is not particularly limited as long as it efficiently absorbs light of 300nm to 400nm in wavelength. A suitable example may be a benzophenone ultraviolet absorbent such as (a) 2-hydroxybenzophenone-4-diglycerylether, (b) 2, 2'-dihydroxybenzophenone-4-diglycerylether, (c) 2-hydroxybenzophenone-4' 4-diglycerylether, (d) 2, 2'-dihydroxybenzophenone-4, 4'-bisglycerylether, (e) 2-hydroxy-4-methoxy-5-sulfobenzophenone sodium salt, (f) 2,2'-dihydroxy-4,4'-dimethoxy-5-sulfobenzophenone sodium salt, (g) 2,2'-dihydroxy-4-methoxybenzophenone-5-sulfonic acid, (h) 2-(2'-hydroxy-4'-methoxy-5'-sulfophenyl) benzotriazole sodium salt, or (i) 2-(2'-hydroxy-4'-butoxy-5'-sulfophenyl) benzotriazole sodium salt.

Among these, a nonionic benzophenone absorbent, such as (a) through (d) are particularly preferable.

The amount of the chelate agent and/or ultraviolet absorbent is generally 0.001 mass % to 10 mass %, preferably 0.01 mass % to 5 mass % with respect to the solid content of the water-absorbent resin. If the amount is more than 10 mass %, the obtained effect does not increase as much as the increase in amount. Further, the absorbency decreases and more group VIII transition metal ions are required. On the other hand, if the amount is less than 0.001 mass %, the durability of water-absorbent resin does not decrease.

The following explains a drying process. The drying process is a process for drying the hydrous gelatinous polymer. The drying method is not particularly limited, and conventional drying methods using a ventilation band-dryer, a rotation-type ventilation drier, a stirring drier, a fluidized-bed dryer, or a vibrational fluidized dryer are suitable.

Further, it is more preferable to carry out high-humidity drying using these driers. In the high-humidity drying, the target material is caused to be in contact with gas which is 50°C to 100°C in dewpoint and at least contains vapor, by using the foregoing driers.

Further, the temperature range for drying the hydrous gelatinous polymer may be used for the range of atmosphere temperature or (material) temperature of the hydrous gelatinous polymer. It is more preferably to use this temperature range as the material temperature of the hydrous gelatinous polymer.

The atmosphere temperature indicates a temperature of hot air, and the material temperature indicates a temperature of the hydrous gelatinous polymer which has been dried in the form of a 40mm lamination. The material temperature is measured by a temperature sensor which is inserted in the vicinity of the center of the lamination in the thickness direction. With the foregoing drying methods, it is possible to effectively decrease the dust amount of the resulting water-absorbent resin.

The temperature for drying the hydrous gelatinous polymer is preferably 80°C to 150°C, more preferably 100°C to 145°C, further preferably 120°C to 140°C. When the drying temperature is less than 80°C, a longer time is required for drying and to reduce the residual monomer. This is not preferable. Further, when the drying temperature is more than 150°C, it causes bumping of the water inside the hydrous gelatinous polymers. As a result, the shapes of the dried water-absorbent resin particles become uneven, which may decrease display effect.

Further, in the process of drying the hydrous gelatinous polymer, a rapid drying of the adhesion block agent on the cross-section surface of the gel may facilitate generation of dust, and therefore may increase dust amount. For this reason, the drying has to be performed in consideration of prevention of such a sudden increase in temperature. Examples include a stepwise drying method using a band drier or a method using a rotary-type through flow drier, which is a rotation-type ventilation drier provided by Okawara Seisakusho, or a high-humidity drying.

This has an effect of decreasing the amount of dust, which is an amount of excessively-small-diameter particles not more than 10$\mu$m in particle diameter with respect to the whole product amount of water-absorbent resin. For example, the temperature at the first stage may be not less than 10°C but less than 120°C, preferably not less than 30°C but less than 115°C, more preferably not less than 50°C but less than 110°C. The drying time at the first stage is preferably not less than 10 minutes but not more than 5 hours, more preferably not less than 10 minutes but not more than 2 hours, further preferably not less than 10 minutes but not more than 1.5 hours.

Further the temperature at the second stage may be not less than 120°C but less than 180°C, preferably not less than 130°C but less than 175°C, more preferably not less than 140°C but less than 175°C. The drying time at the second stage is preferably not less than 10 minutes but not more than 5 hours, more preferably not less than 10 minutes but not more than 2 hours.

The hydrous gelatinous polymer is thus dried under two-stages of temperature, and since the drying is first carried out under a temperature less than 120 °C, it is possible to prevent the water inside the hydrous gelatinous polymer to be suddenly bumped when the temperature is further raised at the second stage. In this manner immune to bumping of water, deformation of the resulting water-absorbent resin does not occur. With this security the drying can be carried out under high temperature: not less than 120°C but less than 180°C. The shapes of resulting water-absorbent resin particles are even, and superior in display effect.

Note that, if the temperature at the first stage is at or greater than 120 °C, the amount of dust in the resulting water-absorbent resin may increase. Further, if the temperature at the second stage is less than 120 °C, the drying has to be carried out for a longer duration. Meanwhile, if the temperature at the second stage is at or greater than 180 °C, the water inside the hydrous gelatinous polymer may be suddenly bumped. This can cause deformation of the resulting water-absorbent resin particles, and the display effect may decrease.

Though it depends on the processing amount of the hydrous gelatinous polymer, the rotation number of the drum is preferably 5min$^{-1}$ to 20min$^{-1}$ when the rotary-type through flow drier, which is a rotation-type ventilation drier provided by Okawara Seisakusho or the like is used.

The wind speed is preferably in a range of 0.5m/s to 20m/s. The temperature of hot air is preferably in a range of 150°C to 200°C. By using the rotary-type through flow drier, it is possible to continuously increase the temperature, and therefore the dust amount after drying the hydrous gelatinous polymer may be reduced.

Further, the moisture content of the dried water-absorbent resin is preferably 5 mass % to 30 mass %, more preferably 10 mass % to 25 mass %, further preferably 15 mass % to 25 mass %. To decrease the moisture content to be lower than 5 mass %, the drying has to be carried on for a relatively long time, which is not preferable. On the other hand, when the moisture content is more than 30 mass %, the water-absorbent resin particles tend to adhere to each other after they are conserved for a long duration of time. Also, the water content of water-absorbent resin increases, meaning that the resin absorbs less amount of liquid, which is not preferable.

The content of residual monomer of the water-absorbent resin is preferably 0 mass ppm to 300 mass ppm, more preferably 0 mass ppm to 200 mass ppm, further preferably 0 mass ppm to 100 mass ppm. If the content is more than 300 mass ppm, it may decrease hygiene of the water-absorbent resin or may cause odor, which is not preferable. The method of measuring the residual monomer is explained later in "Example".

The mass average particle diameter of the water-absorbent resin according to the present invention is preferably 2mm to 10mm, more preferably 2.5mm to 9.0mm, further preferably 3.0mm to 8.0mm. If the mass average particle diameter is less than 2mm, the content of the residual monomer may increase, which is not preferable. On the other hand, when the mass average particle diameter is more than 10mm, the moisture content increases, and the water-absorbent resin particles tend to adhere to each other after they are conserved for a long duration of time. Also, the drying has to be carried on for a relatively long time, which is not preferable.

The logarithm standard deviation of the water-absorbent resin according to the present invention is preferably 0 to 0.25, more preferably 0 to 0.23, further preferably 0 to 0.20. This range of logarithm standard deviation indicates significant sharpness of the particle size distribution of the particulate hydrous gelatinous. That is, the qualities of particles are even.

In the present invention, the particulate water-absorbent resin may be crosslinked in the vicinity of the surface. In this case, the absorbency of the water-absorbent resin under load increases. The surface crosslinking is carried out by a crosslinking agent which reacts with a functional group, such as an acidic group, in the water-absorbent resin. The crosslinking agent may be selected from any-publicly known agents for such a purpose.

Examples of the surface cross-linking agent include multivalent alcohol compounds, such as ethyleneglycol, diethyleneglycol, propyleneglycol, triethylene glycol, tetraethyleneglycol, polyethyleneglycol, 1,3-propanediol, dipropyleneglycol, 2,2,4-trimethyl-1,3-pentanediol, polypropyleneglycol, glycerin, polyglycerin, 2-butene-1,4-diol, 1,4-butanediol, 1,5-pentanediol, 1,6-hexanediol, 1,2-cyclohexanedimethanol, 1,2-cyclohexanediol, trimethylolpropane, diethanolamine, triethanolamine, polyoxypropylene, oxyethylene-oxypropylene block copolymer, pentaerythritol, and sorbitol; multivalent epoxy compounds, such as ethyleneglycol diglycidyl ether, (poly, di) glycerol polyglycidyl ether, polypropyleneglycol diglycidyl ether, polypropyleneglycol diglycidyl ether, or glycidol; multivalent amine compounds, such as ethylenediamine, diethylenetriamine, triethylenetetramine, tetraethylenepentamine, pentaethylenehexamine, polyethylene-imine or the organic/inorganic salt thereof (aziridinium salt etc.); multivalent isocyanate compounds, such as 2,4-trilene diisocyanate or hexamethylene diisocyanate; multivalent oxazoline compounds, such as 1,2-ethylenebisoxazoline; alkylene carbonate compounds, such as 1,3-dioxolan-2-one, 4-methyl-1, 3-dioxolan-2-one, 4,5-dimethyl-1, 3- dioxolan-2-one, 4,4-dimethyl-1, 3- dioxolan-2-one, 4-ethyl-1, 3-dioxolan-2-one, 4-hydroxymethyl-1, 3- dioxolan-2-one, 1,3-dioxolan-2-one, 4-methyl-1,3-dioxane-2-one, 4,6-dimethyl-1, 3- dioxane-2-one, 1,3-dioxopane-2-one; haloepoxy compounds, such as epichloro-hydrin, epibromohydrin, or $\alpha$ -methylepichlorohydrin, and the multivalent amine adducts (eg. *Hercules* natural sponge®; polyvalent metal compounds, such as hydroxide and chloride compound of zinc, calcium, magnesium, alminium, iron, and zirconium.

Among these, multivalent alcohol compounds, multivalent epoxy compounds, multivalent amine compounds, their salts, and alkylene carbonate compounds are particularly preferable. One or plural kinds of the surface crosslinking agent are used according to circumstances.

The amount of the surface crosslinking agent is preferably 0.001 mass % to 10 mass %, more preferably 0.01 mass % to 5 mass %, with respect to 100 mass % of the water absorbent resin.

The heating may be carried out by using a drier or an oven. Examples of the drier/oven includes a thin stirring drier, a rotary drier, a disk drier, a fluidized-bed drier, a flash drier, an infrared drier, and a vibrational fluidized dryer. The heating temperature is preferably 40 °C to 250 °C, more preferably 90 °C to 230 °C, further preferably 120 °C to 220 °C. The heating time is preferably 1 minute to 120 minutes, more preferably 10 minutes to 60 minutes.

The water absorbent resin particles 2mm to 10mm in mass average particle diameter containing particles with 4 - 12 planes, which are obtained by drying the hydrous gelatinous polymer 3mm to 10mm in mass average particle diameter containing particles with 4-12 planes, may be crushed, classified, and granulated to be serve as uneven water-absorbent resin grains.

The water-absorbent resin produced by the method according to the present invention may be mixed with inorganic particles such as oxide titan, silicon dioxide, or activated carbon; organic particles such as polymethyl methacrylate; hydrophilic fiber such as pulp; synthetic fiber such as polyethylene fiber or polypropylene fiber; or surfactant such as polyethyleneglycol, or polyoxyethylenesorbitanmonostearate. These materials may be added to the water-absorbent resin particles during or after the manufacturing process.

Note that, the foregoing method is only one of the examples of manufacturing the new-type water-absorbent resin according to the present invention; however, the method produces the water-absorbent resin of the present application most efficiently. The water-absorbent resin can also be manufactured by other methods, such as the following methods 2 and 3.

(Production Method 2)

The hydrous gel is cut before or after the drying process. Then, only the particles satisfying the condition of the present invention are collected. That is, if the hydrous gelatinous particles fall out of the range of 3mm to 10mm in mass average particle diameter, or the dried particles fall out of the range of 3mm to 10mm in mass average particle diameter, or the content of the particles with 4 - 12 planes falls out of the ratio range of 50 mass % to 100 mass %.

The collecting of the particles satisfying the condition of the present invention may be carried out, for example, by a screener or a rotation screener (both described later). Also, the collecting may be carried out by a rotation screener (described later).

(Production Method 3)

The hydrous gelatinous polymer 3mm to 10mm in mass average particle diameter constituted of particles with 4 - 12 planes are produced by polymerization of the foregoing monomer, and the hydrous gelatinous polymer 3mm to 10mm in mass average particle diameter constituted of particles with 4 - 12 planes are then taken out from the polymerization container without being cut into pieces.

(2. Cutting device)

The cutting device according to the present invention serves to cut a sheet of the hydrous gelatinous polymer (gel sheet). The cutting device comprising at least one vertical-cutting blade for vertically cutting the hydrous gelatinous polymer (cutting the width of polymer gel); at least one horizontal-cutting spinning blade and fixed blade for horizontally cutting the hydrous gelatinous polymer (cutting the length of polymer gel); and at least one diffusing means for spraying/diffusing an adhesion block agent onto the hydrous gelatinous polymer or onto the vertical-cutting blade, the horizontal-cutting spinning blade and/or the fixed blade. By using this cutting device in the cutting process, the resulting hydrous gelatinous polymer mainly contains particles with 6 smooth planes, and the surface is partly coated with adhesion block agent.

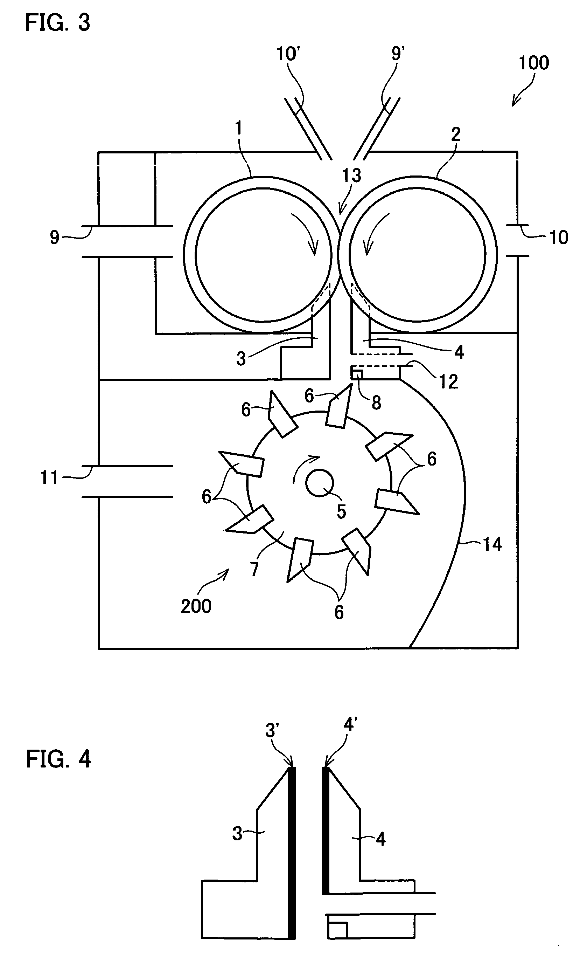

The following explains the cutting device with reference to Figures 3 through 7. Figure 3 is a vertical cross-sectional view showing a structure of a cutting device according to the present embodiment. As shown in the figure, a cutting device 100 includes vertical-cutting blades (roll cutter) 1 and 2, scrapers 3 and 4, a horizontal-cutting spinning blade 200, a fixed blade 8, spraying and/or diffusing devices (spraying and/or diffusing means) 9, 10, 11 and 12, a gel sheet inlet 13, and a casing 14. The spraying and/or diffusing devices (spraying and/or diffusing means) 9' and 10' may be provided if required. The horizontal-cutting spinning blade 200 includes a vertical cutting (a movement of cutting the length of the gel) rotation axis 5, a blade 6, and a horizontal-cutting spinning body 7. The horizontal-cutting spinning blade 200 and a fixed blade 8 constitute a clipping cutter device.

The spraying and/or diffusing devices (spraying and/or diffusing means) 9, 10, 11, 12, 9' and 10' serve to spray/diffuse the adhesion block agent onto the fixed blade 8, the blade 6, the vertical-cutting blades (roll cutter) 1 and 2, and/or the hydrous gelatinous polymer (not shown). With the spraying and/or diffusing devices (spraying and/or diffusing means) 9, 10, 11, 12, 9' and 10', the cutting device 100 efficiently coats the hydrous gelatinous polymer with an adhesion block agent. On this account, the hydrous gelatinous polymer particles resulted from the cutting process are not adhered to each other.

More specifically, the adhesion block agent is applied from the spraying and/or diffusing devices (spraying and/or diffusing means) 9, 10, 11, 12, 9' and 10' onto the fixed blade 8, the blade 6, the vertical-cutting blades (roll cutter) 1 and 2, and/or the hydrous gelatinous polymer. As a result, the surfaces of the fixed blade 8, the blade 6, the vertical-cutting blades (roll cutter) 1 and 2 and/or the hydrous gelatinous polymer are partly coated. On this account, the hydrous gelatinous polymer particles resulted from the cutting process are securely prevented from adhering to each other.

The method of supplying the adhesion block agent to the spraying and/or diffusing devices (spraying and/or diffusing means) 9, 10, 11, 12, 9' and 10' is not particularly limited. For example, a conventional pump or the like (not shown) may be used. Further, though the example shown in Figure 3 uses four spraying and/or diffusing devices (spraying and/or diffusing means) 9, 10, 11, 12, 9' and 10' the number of spraying and/or diffusing devices (spraying and/or diffusing means) is not limited. A larger or smaller number of spraying and/or diffusing devices (spraying and/or diffusing means) may be used according to required effect, for example, desired efficiency.

The locations of the spraying and/or diffusing devices (spraying and/or diffusing means) 9, 10, 11, 12, 9' and 10' are not particularly limited; however, it is necessary to locate them with assurance of the function of spraying/diffusion the adhesion block agent onto the hydrous gelatinous polymer, the fixed blade 8, the blade 6, and the vertical-cutting blades (roll cutter) 1 and 2, so as to ensure the desired efficiency in coating the hydrous polymer gel with the adhesion block agent. In the structure of Figure 3, the adhesion block agent splayed/diffused from the spraying and/or diffusing devices (spraying and/or diffusing means) 9 and 10 is mostly adhered to the vertical-cutting blades (roll cutter) 1 and 2, and then is adhered onto the hydrous gelatinous polymer when the hydrous gelatinous polymer is cut into pieces as it is inserted into the gel sheet inlet 13 and travels between the vertical-cutting blades (roll cutter) 1 and 2, or before or after the hydrous gelatinous polymer passes through the vertical-cutting blades (roll cutter) 1 and 2. Consequently, the hydrous gelatinous polymer is coated with the adhesion block agent.

Further, the most of adhesion block agent splayed/diffused from the spraying and/or diffusing devices (spraying and/or diffusing means) 9 and 10 is directly adhered onto the hydrous gelatinous having been cut into pieces by the vertical-cutting blades (roll cutter) 1 and 2. Consequently, the hydrous gelatinous polymer is coated with the adhesion block agent.

Further, the adhesion block agent splayed/diffused from the spraying and/or diffusing devices (spraying and/or diffusing means) 11 and 12 is mostly adhered to the blade 6, the fixed blade 8, and the horizontal-cutting spinning body 7, and then is transferred mainly from the blade 6 and the fixed blade 8 onto the hydrous gelatinous polymer when the hydrous gelatinous polymer is being cut into pieces by the blade 6 and the fixed blade 8, or before or after the hydrous gelatinous polymer is cut into pieces by the blade 6 and the fixed blade 8. Consequently, the hydrous gelatinous polymer is coated with the adhesion block agent.

Further, the adhesion block agent splayed/diffused from the spraying and/or diffusing devices (spraying and/or diffusing means) 9, 10, 11, 12, 9' and 10' is also adhered to the components of the cutting device 100, such as the vertical-cutting blades (roll cutter) 1 and 2, the blade 6, or the horizontal-cutting spinning body 7. This prevents the hydrous gelatinous polymer from adhering to the components. On this account, the functions of the components, such as a cutting function, can be maintained.

The spraying and/or diffusing devices (spraying and/or diffusing means) 9, 10, 11, 12, 9' and 10' may be realized by any publicly-known spraying/diffusing means, for example, an air-mixing type spraying device such as an atomizer, 1-fluid nozzle or 2-fluid nozzle; or a liquid injecting pump. The type of the spraying/diffusing means of the present invention is not particularly limited.

The horizontal-cutting (cutting the length of the gel) rotation axis 5 serves to rotate the horizontal-cutting spinning body 7. The force for rotating the horizontal-cutting (cutting the length of the gel) rotation axis 5 is not particularly limited. For example, the horizontal-cutting (cutting the length of the gel) rotation axis 5 may be rotated manually or by publicly-known means such as a motor. In Figure 3, the rotation direction of the horizontal cutting (cutting the length of the gel) rotation axis 5 is clockwise, but the present invention is not limited to this.

The horizontal-cutting spinning body 7 includes the blade 6 on its periphery section, and moves along with the rotation of the horizontal cutting (cutting the length of the gel) rotation axis 5. In Figure 3, the cutting device 100 includes two types of rotation blades: the horizontal-cutting spinning blade 200 constituted of the horizontal-cutting (cutting the length of the gel) rotation axis 5, the blade 6, and the horizontal-cutting spinning body 7; and the vertical-cutting blades (roll cutter) 1 and 2.

The ratio of the peripheral velocity of the vertical-cutting blades (roll cutter) 1 and 2 to that of the horizontal-cutting spinning blade 200 is determined depending on the length of the hydrous gelatinous polymer. The peripheral velocity of the horizontal-cutting spinning blade 200 is set to an optimal value in consideration of the temperature, or the rigidity of a strand polymer resulted from the vertical cutting of the hydrous gelatinous polymer. If the peripheral speed is excessively low, generally, the cutting operation may not be properly performed, and the smoothness on the cross-section of the polymer may decrease. On the other hand, if the peripheral speed is excessively high, the pieces resulted from the cutting of the polymer tend to have varied sizes, and the uniformity of the polymer particles in size cannot be ensured.

The blade 6 serves to horizontally cut the hydrous gelatinous polymer having been cut by the vertical-cutting blades (roll cutter) 1 and 2, by setting the hydrous gelatinous polymer between itself and the fixed blade 8. The location and the number of the blade 6 are not particularly limited, but it is preferable to provide 1 to 10 of the blades 6 evenly aligned. The exact number is however determined in consideration of the cutting speed and the cutting length. In terms of the balance, an even number is preferable, but an odd number is also acceptable.

The fixed blade 8 serves to horizontally cut the hydrous gelatinous polymer having been cut by the vertical-cutting blades (roll cutter) 1 and 2, by setting the hydrous gelatinous polymer between itself and the blade 6.

The clearance between the fixed blade 8 and the blade 6 is not particularly limited, but preferably 0.005mm to 0.05mm, more preferably 0.01mm to 0.04mm, further preferably 0.01mm to 0.03mm.

If the clearance is less than 0.005, there is not enough space between the fixed blade 8 and the blade 6, and therefore the two blades come in contact with each other. This causes abrasion or damage of the blades. On the other hand, a clearance larger than 0.05mm gives an excessive large space between them, causing some difficulties in cutting performance. The vertical-cutting blades (roll cutter) 1 and 2 serve to vertically cut (cut the length) the hydrous gelatinous polymer as the hydrous gelatinous polymer is supplied through the gel sheet inlet 13. The respective diameters of the vertical-cutting blades (roll cutter) 1 and 2 are typically determined depending on the width of sheet subjected to the cutting. To cut a sheet having a large width, the diameter of the blade needs to be increased so as to maintain the accuracy of the cutting width and suppress the flexure of the blade.

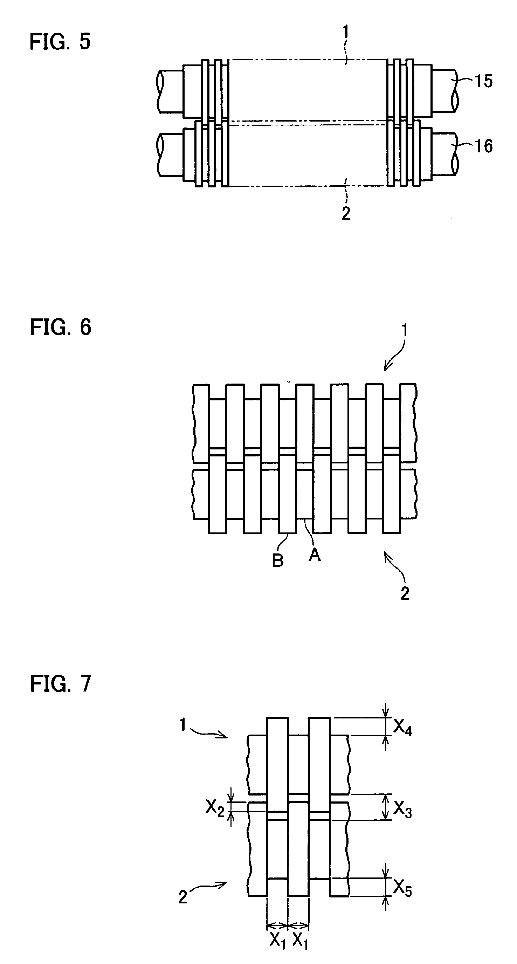

Figure 5 is an explanation view showing an upper view of the vertical-cutting blades (roll cutter) 1 and 2. Figure 6 is an explanatory view showing a portion where the engagement between vertical-cutting blades (roll cutter) 1 and 2 is established. Figure 7 is a magnified view of the two blades engaged with each other. As shown in Figures 5, 6 and 7, the surface of each blade of the vertical-cutting blades (roll cutter) 1 and 2 has depressions and projections so as to allow them to be engaged with each other. The rotation axes 15 and 16 serve to apply rotation force to the vertical-cutting blades (roll cutter) 1 and 2. These engaged vertical-cutting blades (roll cutter) 1 and 2 have the same size, and rotate at the same speed into a certain direction to maintain the engagement. The blades to be engaged may have desired widths, depths and heights according to the desired size of the vertical-cutting blades (roll cutter) 1 and 2. For example, the blades shown in Figure 7 are set to have a width (X1) of 2mm to 10mm, a depression (X4) of 10mm to 15mm, and a projection (X5) of 10mm to 15mm. In this case, the clearance (X3) is generally set to 10mm to 25mm even when the depression and the projection are engaged with each other to the deepest extent. With this arrangement the pieces of the hydrous gelatinous polymer resulting from the cutting have enough space to pass through.

With such an arrangement on the surface of each blade of the vertical-cutting blades (roll cutter) 1 and 2, the two vertical blades (roll cutter) 1 and 2 are rotated by the two rotation axes 15 and 16, and the vertical blades 1 and 2 draw in the hydrous gelatinous polymer supplied from the gel sheet inlet 13 and cut the polymer into pieces. The vertical-cutting blades 1 and 2 then convey the pieces downward. In this way, the hydrous gelatinous polymer may be cut along the length direction.

This allows the hydrous gelatinous polymer to be cut into a strand strip of sheet.

Note that, by continuously supplying the hydrous gelatinous polymer to the vertical-cutting blades (roll cutter) 1 and 2 by, for example, continuously taking out the hydrous gelatinous polymer from one end of a movable supporter, which may be realized by an endless belt or the like, so that the hydrous gelatinous polymer is conveyed into the blades of the vertical-cutting blades (roll cutter) 1 and 2, the entire process can be continuously performed, and the production efficiency increases. Next, the following explains the method of horizontally cutting the strand strip gel sheet. The gel sheet having been cut into a strand strip or the like by the vertical-cutting blade (roll cutter) 1 or 2 is removed from the periphery of the inner blade (denoted by "A" in Figure 6) of the vertical-cutting blade (roll cutter) 1 or 2 by the upper edge of the scraper 3 or 4. The strand-shaped gel is then conveyed downward between the scrapers 3 and 4 and reaches the fixed blade 8 under the scraper 4. It is not likely that the hydrous gelatinous polymer strand is adhered to the outer blades (denoted by "B" in Figure 6) of the vertical-cutting blades (roll cutter) 1 and 2.

Note that, the scrapers 3 and 4 are preferably coated or made of a material not to be adhered to the resulting strand shaped hydrous gelatinous polymer, which may cause clogging. Coating is more desirable in terms of cost (Figure 4). More specifically, as shown in Figure 4, it is preferable to coat the vertical-cutting blades (roll cutter) 1 and 2, the horizontal-cutting spinning blade 200, and 3' and 4' of the scrapers 3 and 4, which are to be in contact with the strand shaped hydrous gelatinous polymer. The coating may be performed by plating, flame spray coating, or vapor deposition using a metal material, or heat treatment using a resin material. The way of coating is however not limited.

A resin coating material may be non-adhesion paint or the lubricative paint. Examples include fluorinated paint such as polytetrafluoroethylene (4 fluoride, PTFE) paint, tetrafluoroethylene hexafluoropropylene copolymer paint (FEP), tetrafluoroethylene perfluoroalkylvinylether copolymer (PFA) paint, fluoroplastic denatured paint (one coat enamel), ethylene tetrafluoroethylene copolymer (ETFE); silicone paint, polyvinyl chloride paint and polyamide paint.

Examples of the fluoroplastic denatured paint (one coat enamel) include Du-Pont 420-105 (ONE COAT GRAY: product of Du-Pont Kabushiki Kaisha). The components and their contents of this paint are 10-15wt% of fluoroplastic, 10-15wt% of organic binder resin, 15-25wt% of methylisobutylketone, 1-5wt% of diacetone alcohol, 50-55wt% of N-methyl pyrrolidone, and 5-10wt% of titanium oxide.

Further, the metal material is not limited as long as it does not adhere to the hydrous gelatinous polymer. Examples of metal plating include Kanigen plating which carries out electroless chemical processing of nickel and phosphorus, Kanifreon plating in which eutectoid particles of fluoroplastic or the like are evenly dispersed in a Kanigen plating layer, ceramic Kanigen plating in which eutectoid particles of metal oxide, carbide or the like are dispersed in a Kanigen plating layer, and titan. Examples of titan plating include vapor deposition, sputtering, or ion plating using TiN, TiCN, TiAlN, Crn etc.

Since the abrasion needs to be taken into account, it is preferable to use titan coating ensuring high durability for the vertical-cutting blades (roll cutter) 1 and 2, the blade 6 and the blade portion of the fixed blade 8. For the parts the abrasion is not concerned such as a part of the scrapers 3 and 4, the boulder of the spinning blade, or casing, it is preferable to use a fluorocarbon paint.

The strand strip having reached the fixed blade 8 further travels downward, and is then split by the cutting force between the blade 6 and the fixed blade 8 into gel particles mainly containing angular-shaped particles with 6 smooth planes. The particles are preferably 3mm to 10mm in mass average particle diameter, and have the particle diameter distribution of not more than 0.25.

The shape of the cross-sectional surface of the gel strip depends on the machine size of the assembled vertical-cutting blades (roll cutter) 1 and 2, namely the widths (X1) of depression/projection of the blade, depth (X4) of depression, height (X5) of projection, depth (X2) of engagement, and rotation speed of the vertical-cutting blades (roll cutter) 1 and 2. Further, by adjusting the rotation speed of the vertical-cutting blades (roll cutter) 1 and 2, the rotation speed of the horizontal-cutting spinning body 7 which corresponds to the cutting length, and the number of blades to be provided on the horizontal-cutting spinning body 7, it is possible to determine the mass average particle diameter of the hydrous gelatinous polymer.

As described, the scrapers 3 and 4 serve to remove the hydrous gelatinous polymer from the periphery of the inner blades (denoted by "A" in Figure 6) of the vertical-cutting blades (roll cutter) 1 and 2. The gel sheet inlet 13 serves to supply a sheet-shaped hydrous gelatinous polymer, which was produced through stationary polymerization, to the vertical-cutting blades (roll cutter) 1 and 2. The casing 14 serves to block scattering of the hydrous gelatinous polymer. The casing 14 may have any desired shape.

Note that, the fixed blade 8 is described above to be provided on the lower end of the scraper 4 so as to perform horizontal cutting, but the number or location of the fixed blade 8 is not limited to those above. The fixed blade 8 may perform both horizontal and vertical cutting.

In the cutting device 100, it is preferable that the temperature at the time of the cutting process of the hydrous gelatinous polymer be as low as possible and the tension of the gel sheet be controlled. With this arrangement, the cutting efficiency increases and the polymer may be cut into even certain shapes. The temperature of the hydrous gelatinous polymer in the cutting process is preferably at or lower than 70°C, more preferably at or lower than 60°C, further preferably at or lower than 50°C, still further preferably at or lower than 40°C, yet further preferably at or lower than 30°C, most preferably at or lower than 20°C.

The width of the gel sheet is generally narrowed as it is tense by a pulling force of the vertical-cutting blade (roll cutter) 1 or 2. That is, the tension of gel sheet can be indirectly controlled by adjusting the width of gel sheet. The width of gel sheet changes depending on the cutting speed or the type of transfer means which carries the sheet to the cutting device. It is however preferable that the width of the tense gel sheet be at least 90% of the general width, i.e., the width before the gel sheet is transferred to the cutting device. If the gel sheet is too tense, the gel sheet is greatly deformed before subjected to cutting process. Consequently the cutting width of the gel sheet varies. This is not desirable.

The temperature of the hydrous gelatinous polymer can be lowered, for example, by carrying out some kind of sufficient cooling in the polymerization process, by compulsively cooling the hydrous gelatinous polymer by cool blast or the like before the cutting process and/or by using a cooling belt. One example of the method using a cooling belt may be carried out by transferring the gel sheet upward on an endless belt (e.g. endless belt provided by SUS) horizontally placed, and sending cool blast from a lower (inner) portion of the belt.

Further, by carrying out the cutting process under dry air or cool blast (preferably not more than 25°C), it is possible to further reduce stickiness of the hydrous gelatinous polymer or adhesion of the hydrous gelatinous polymer particles cut into pieces. One specific example of the cutting device is a cubing pelletizing machine PM-300 (TANAKA IRON WORKS CO., LTD).

[3. Mixer]

Further preferably, the hydrous gelatinous polymer particles resulted from the cutting process are mixed by a mixing device so as to ensure uniform coating of their surfaces with the adhesion block agent. By supplying extra adhesion block agent in the mixing device, the adhesion blocking property of the hydrous gelatinous polymer further increases. The mixer may also be used for adding a chelate agent and/or ultraviolet absorbent.

The mixing device may be realized by any publicly-known device, such as a Lödige mixer (MATSUBO Corporation.), a cylindrical mixer, a screw mixer, a screw extruder, a turbulizer, a nauta mixer, a V-shaped mixer, a ribbon blender, a double-arm kneader, a flow mixer, an air current mixer, a rotary disc mixer, a roll mixer, or a convolution mixer. Either of a continuous-type mixer or a batch-type mixer may be used. However, in order to ensure the adhesion blocking property, to make the spraying amount of the adhesion block agent even, and to make the retention time of the agent in the device even, a batch-type Lödige mixer is more preferable, and a Lödige mixer is most preferred among the listed devices.

In the case of using a batch-type Lödige mixer, the following mixing method may be used, for example. A predetermined amount of the hydrous gelatinous polymer having been cut into pieces by the cutting device 100 is first supplied to the Lödige mixer. Then an adhesion block agent is sprayed into the mixer to be mixed with the hydrous gelatinous polymer.

The hydrous gelatinous polymer may be subjected to the previous mixing for a predetermined time before the adhesion block agent is sprayed into the gel. Further, after spraying in all of the adhesion block agent, the mixing is carried on further if necessary (post mixing). By carrying out the previous mixing, the hydrous gel is crushed and therefore may be well-coated with the adhesion block agent.

In the mixing, the blade is preferably rotated at a speed of 100min$^{-1}$ to 200min$^{-1}$, and the side chopper is preferably rotated at a speed of 1000min$^{-1}$ to 4000min$^{-1}$.

An appropriate mixing time varies depending on the capacity of the Lödige mixer and the filling ratio of the material, but the previous mixing is preferably performed for 0 second to less than 2 minutes, the main mixing is preferably performed for 10 seconds to less than 3 minutes, and the post mixing is preferably performed for 0 second to less than 5 minutes.

A preferable material is a materal which can prevent the hydrous gelatinous polymer from being adhered to the internal wall of the device. Coating is more desirable in terms of cost.

The coating may be performed by plating, flame spray coating, or vapor deposition using a metal material, or heat treatment using a resin material. The way of coating is however not limited.

A resin coating material may be non-adhesion paint or the lubricative paint. Examples include fluorinated paint such as polytetrafluoroethylene (4 fluoride, PTFE) paint, tetrafluoroethylene hexafluoropropylene copolymer paint (FEP), tetrafluoroethylene perfluoroalkylvinylether copolymer (PFA) paint, fluoroplastic denatured paint (one coat enamel), tetrafluoroethylene ethylene copolymer (ETFE); silicone paint, polyvinyl chloride paint and polyamide paint.

Examples of the fluoroplastic denatured paint (one coat enamel) include Du-Pont 420-105 (ONE COAT GRAY: provided by DU-PONT Kabushiki Kaisha). The components and their contents of this paint are 10-15wt% of fluoroplastic, 10-15wt% of organic binder resin, 15-25wt% of methylisobutylketone, 1-5wt% of diacetone alcohol, 50-55wt% of N-methyl pyrrolidone, and 5-10wt% of titanium oxide.

Further, the metal material is not limited as long as it does not adhere to the hydrous gelatinous polymer. Examples of metal plating include Kanigen plating which carries out electroless chemical processing of nickel and phosphorus, Kanifreon plating in which eutectoid particles of fluoroplastic or the like are evenly dispersed in a Kanigen plating layer, ceramic Kanigen plating in which eutectoid particles of metal oxide, carbide or the like are dispersed in a Kanigen plating layer, and titan. Examples of titan plating include vapor deposition, sputtering, or ion plating using TiN, TiCN, TiAlN, Crn etc.

The mixing device may also be used for mixing of the surface crosslinking agent used for the crosslinking of the water-absorbent resin. The extra adhesion block agent is supplied by being sprayed/diffused into the mixer, but this can be performed by any publicly known spraying/diffusing means such as an air-mixing type spraying device such as an atomizer, 1-fluid nozzle or 2-fluid nozzle; a liquid injecting pump; a brush; or a roller.

(4. Water-absorbent resin)

A water-absorbent resin according to one embodiment of the present embodiment is produced by polymerizing an ethylenically-unsaturated monomer containing 30-100 mole% acrylic acid and/or acrylate, and cutting the resulting hydrous gelatinous polymer into particles containing particles with 4-12 planes at a ratio of 50 mass % with respect to the mass of the hydrous gelatinous polymer, and drying the particles. The mass average particle diameter of the water-absorbent resin is 2mm to 10mm, and the distribution of logarithm standard deviation is 0-0.25, and the content of residual monomer is 0 mass ppm to 300 mass ppm.

The foregoing acrylic acid and/or acrylate, and the acrylate monomer designate acrylic acid or acrylic water soluble salts. The ethylenically-unsaturated monomer preferably include an acrylate monomer as a main component by which the water absorbency and security of the hydrous gelatinous polymer increases.

Particularly preferable examples of acrylate include alkali metal salts, alkali metal earth salts, ammonium salts, hydroxyl ammonium salts, amine salts and alkyl amine salts of acrylic acid whose neutralization ratio is 30 to 100 mole%, more preferably 50 to 99 mole%. Sodium salt and potassium salt are particularly preferable among the water soluble salts.

When the neutralization ratio is 70 to 100 mole%, polymerization reaction proceeds particularly smoothly, and the yield of the water-absorbent resin increases. One or more kind of these acrylates may be used according to the circumstance.

As described above, the vertical and horizontal cutting of the hydrous gelatinous polymer creates the hydrous gelatinous polymer particles which contains particles with 4-12 planes at a ratio of 50 mass % to 100 mass % with respect to its gross mass. The dried polymer particles are classified so that the particles become 2mm to 10mm in mass average particle diameter, and 0.25 or less in logarithm standard deviation of the particle diameter distribution. The water-absorbent resin is a product of drying the hydrous gelatinous polymer of even-sized particles. Therefore the resin has desirable dry condition.

With significantly small amount of residual monomer, namely at a ratio of 0 mass ppm to 300 mass ppm, the water-absorbent resin has high quality and is broadly free from problems of odor or insanitation.

The water-absorbent resin whose moisture content is 5 mass % to 30 mass % with respect to its gross mass is obtained by drying the hydrous gelatinous polymer having a sufficient moisture content to cause reaction between the residual monomer and a polymerization initiator. The specific content of residual monomer of the water-absorbent resin, namely 0 mass ppm to 300 mass ppm, is thus securely obtained. A water-absorbent resin according to one embodiment of the present invention is obtained by drying a hydrous gelatinous polymer which is polyhedron particles. The surfaces of the water-absorbent resin particles are at least partly coated with an adhesion block agent containing at least one kind of compound classified into multivalent metallic salt, multivalent alcohol or a surfactant.

Such a water-absorbent resin can be produced by the above-described method for manufacturing water-absorbent resin according to the present invention.

The amount of the adhesion block agent sprayed and/or diffused into the particles is preferably in a range of 0.0015 mass % to 35 mass % with respect to the solid content of the water-absorbent resin.

If the spraying and/or diffusing amount is less than 0.0015 mass %, the hydrous gelatinous polymer particles are not fully coated, and the polymer particles are more likely to adhere to each other. Accordingly, the hydrous gelatinous polymer particles tend to aggregate during the drying process, interfering smooth drying. On the other hand, if the spraying and/or diffusing amount is more than 35 mass %, the polymer becomes sticky and difficult to be handled. As well as this, the particles may not be desirably distributed during the drying process. Further, the large amount of adhesion block agent is economically not desirable.

A water-absorbent resin according to one embodiment of the present invention contains particles, each of which has an angular shape with smooth planes, at a ratio of 50 or more mass % with respect to the solid content. The properties of the remaining particles are not particularly limited as long as they are angular-shaped polyhedron particles with smooth planes.

The remaining particles however preferably have 4-12 planes. Further, in terms of superior durability, the water-absorbent resin of the present invention preferably contains chelate agent and/or ultraviolet absorbent.

(5. Screener)

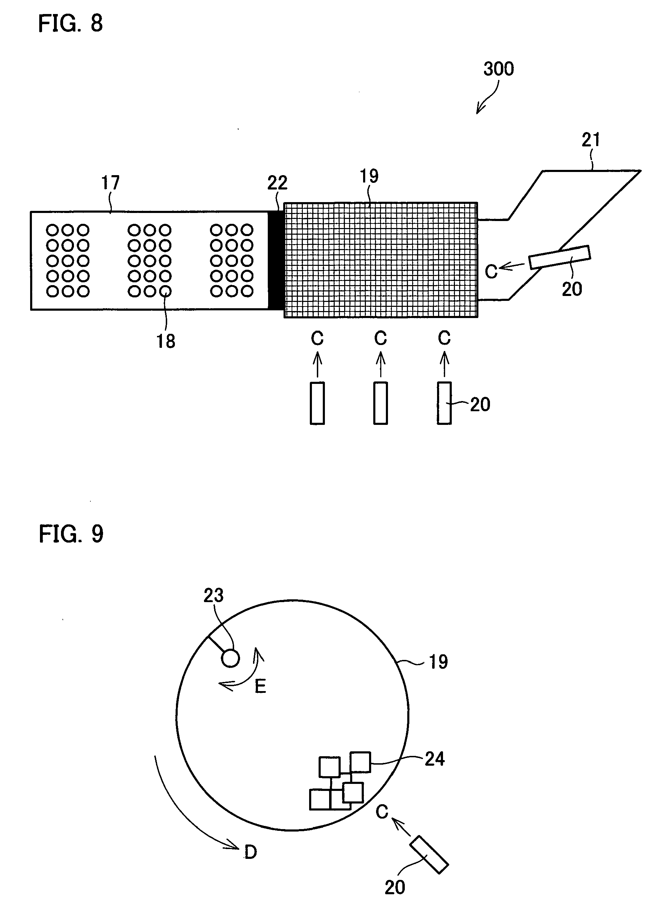

The screener according to the present embodiment is used for screening the water-absorbent resin resulted from the drying process, based on the desired mass average particle diameter. Figure 8 is a schematic view of a lateral view of an example of a rotation screener 300.

The screener 300 includes water-absorbent resin inlet 21 for supplying the water-absorbent resin, a first removing section 19 for removing excessively-small-diameter particles of the water-absorbent resin, a second screening section 17 for removing excessively-large-diameter particles of the water-absorbent resin.

The excessively-small-diameter particles of water absorbent resin are smaller than the mass particle diameter of the dried water-absorbent resin, the particles being able to pass through a mesh or a punching of 0.1mm to 9mm. The excessively-small-diameter particles may be the hydrous gelatinous polymer particles having been cut twice and dried, or may be scrap or dust. The excessively-large-diameter particles are larger than the mass particle diameter of the dried water-absorbent resin, the particles being not able to pass through a mesh or a punching of 2.0mm to 10mm. The excessively-large-diameter particles may be aggregation of two or more particles of the hydrous gelatinous polymer particles, or may be particles resulting from inadequate cutting.

With this cutting process using the cutting device 300, it is possible to adjust the particles to 2mm to 10mm in mass average particle diameter, and to 0 to 0.25 in logarithm standard deviation.

The diameters of the excessively-small-diameter particles of water absorbent resin and the excessively-large-diameter particles of water absorbent resin to be removed may be arbitrary set by changing the diameter of the punching 18 in the second screening section 17 (described below) and changing the diameter of the net of the first screening section 19.

The first screening section 19 and the second screening section 17 are each formed as a cylinder. A cauld 22 is preferably provided between the second screening section 17 and the first screening section 19. The cauld 22 is smaller in internal diameter than the first screening section 19 or the second screening section 17. The second screening section 17 includes a punching (opening section) and one or more gas spray nozzles 20 which are gas spray mechanisms for spraying gas to the water-absorbent resin inside the first screening section 19. The locations of the gas spray nozzles 20 are not limited and may be appropriately determined. By spraying gas from one of the upper face, lateral face, the bottom face and the material supplying inlet of the first screening section 19, the removal of excessively-small-diameter particles is further ensured.

The material supplying inlet 21 is an opening for supplying the water-absorbent resin. The material supplying inlet is adjacent to the first screening section 19, and the water-absorbent resin particles are moved to the first screening section 19 as they are poured from the inlet 21. The first screening section 19 for removing excessively-small-diameter particles of the water-absorbent resin is made of a cylinder net.

The material filling ratio of the first screening section 19 is 5 to 40 vol%, preferably 10 to 30 vol%, more preferably 10 to 20 vol%. If the filling ratio is less than 5 vol%, the absorbent-resin particles may not stay in the first screening section 19 long enough, and the removal of excessively-small-diameter particles may not be sufficient.

On the other hand, if the filling ratio is more than 40 vol%, the volume of the water-absorbent resin in the first screening section 19 is too large, and the excessively-small-diameter particles removing effect may be reduced.

The diameter of the first screening section 19 is not limited, but preferably not less than 2.0 mm but not more than 3.0 mm in the case of producing water-absorbent resin blocks 4.0 mm in mass average particle diameter.

Figure 9 shows a cross-section of the first screening section 19. As shown in the figure, the first screening section 19 is structured to be rotated in the direction indicated by an arrow D. The first screening section 19 is surrounded by the gas spray nozzles 20, which spray gas toward the direction C indicated by an arrow C. The type of gas is not particularly limited but air is preferred in terms of cost. It is preferable to use dry air with a dewpoint of not more than -10°C, more preferably dry air with a dewpoint of not more than -20°C. With its rotating action, the first screening section 19 efficiently sprays gas into the water-absorbent resin 24 contained therein.

The speed (retention period) of the first screening section 19 is 5 to 50 rpm, preferably 10 to 40 rpm, more preferably 10 to 20 rpm. If the speed falls below 10 rpm, the material is not smoothly supplied, and the filling ratio of the water-absorbent resin in the first screening section 19 excessively increases. On the other hand, if the speed exceeds 30 rpm, the material is supplied too quickly, and the filling ratio of the water-absorbent resin in the first screening section 19 excessively decreases.

The gas spray nozzle 20 is not particularly limited, and any publicly-known gas spray mechanism, such as an atomizer, 1-fluid nozzle or a wonder gun may be used. The spraying of gas may be performed either continuously or intermittently.

The first screening section 19 and/or the second screening section 17 may contain a built-in hammer 23, 23a and 23b. The hammer 23, 23a and 23b are structured to vibrate in the direction denoted by an arrow E, and their vibration causes the first screening section 19 and/or the second screening section 17 to vibrate. The vibration has an effect of removing irregular particles caught in the net of the first screening section 19 and/or the second screening section 17, thereby preventing clogging of the first screening section 19 and/or the second screening section 17. To further ensure prevention of clogging, each of the first screening section 19 and the second screening section 17 preferably includes at least one hammer 23, 23a and 23b.

The cauld 22 is preferably provided between the first screening section 19 and the second screening section 17. The cauld 22 is smaller in internal diameter than the first screening section 19, and therefore blocks a small amount of the water-absorbent resin on the way of moving into the second screening section 17. Consequently, the retention time of the water-absorbent resin 24 in the first screening section 19 increases, and the effect of removing the excessively-small-diameter particles is facilitated. By thus ensuring removal of fine particle, the content of excessively-small-diameter particles in the resulting water-absorbent resin is reduced. The resulting resin is therefore superior in display effect.

The second screening section 17 has a plurality of punchings 18. Each of the punchings 18 has a diameter smaller than the preferable mass particle diameter of the water-absorbent resin, so that the excessively-small-diameter particles of the water-absorbent resin to be removed will remain in the second screening section 17.

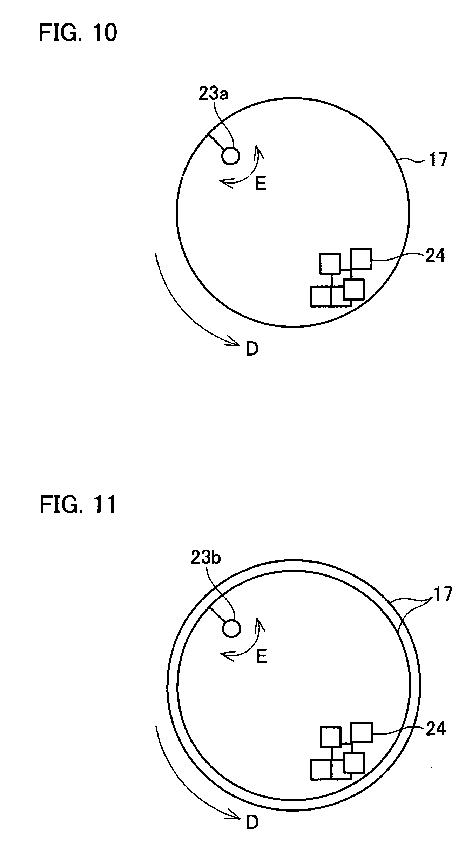

Further, the second screening section 17 may have a structure with a single punching cylinder as shown in Figure 10, or may have a multilayer structure as shown in Figure 11. Figure 8 shows an example of the screening device 300 with a double-screening structure. In this screening device 300, the second screening section 17 has another screening section therein, and the two second screening section 17 are oppositely positioned. On the other hand, the respective punchings 18 (shown by a solid line) of the two second screening section 17 (shown by a broken line) are not opposed to each other. More specifically, the respective punchings 18 are not opposed even partially.

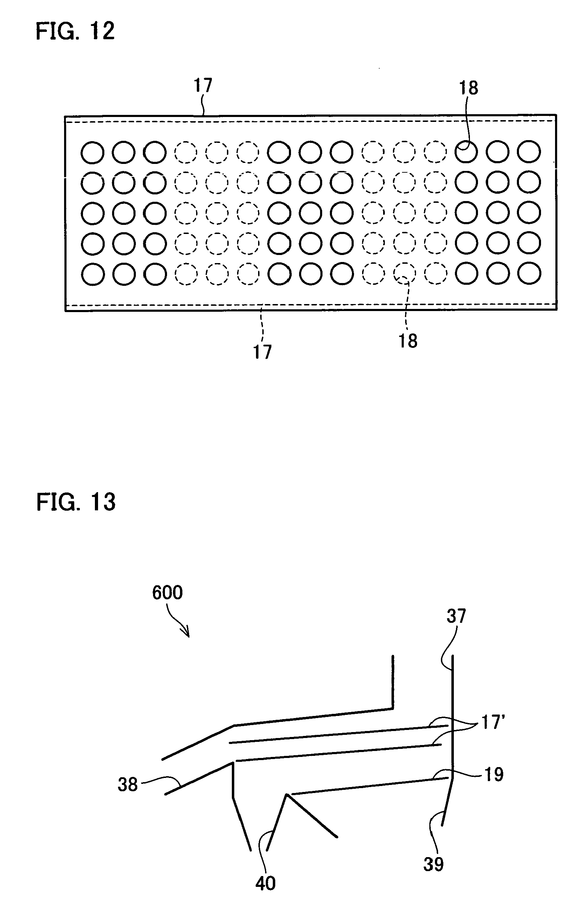

Figure 12 is a schematic projection view showing the screening section with a double-layer structure. As shown in the figure, the punchings 18 of the second screening section 17 are not opposed to the punchings 18 of the other second screening section 17. The gap between the two layers of second screening section 17 is preferably at least 1 times but not more than 2 times, more preferably at least 1 times but not more than 1.8 times the mass average particle diameter of the water-absorbent resin particles so that the water-absorbent resin particles to pass through the punchings 18 while a long-sized particles remain in the second screening section 17. In this way, it is possible to obtain the water-absorbent resin uniform in particle size, which are superior in display effect.

The diameter of the punching 18 is not particularly limited but preferably in a range of 6.0 mm to 8.0 mm in the case of collecting water-absorbent resin blocks 4.0 mm in mass average particle diameter.

In one example structure for obtaining the water-absorbent resin blocks 4mm in mass average particle diameter, the diameter of net of the first screening section 19 of the screening device 300 is set to 2.8mm, and the diameter of each punching 18 of the two layers of second screening section 17 is set to 7mm and 9mm, respectively. The gap between the inner punching and the outer punching is set to 7mm.

Examples of such a rotation screening device includes SPSR-2000, SPSR-1500W, SPSR-1000, SPSR-500, and SPSR-250, each of which has a single layer of second screening section 17; and SPSR-2000w, SPSR-1500w, SPSR-1000w, SPSR-500w, and SPSR-250w, each of which has two layers of second screening section 17 (SPSR series are all product of *Seiwa Tekkosho CO., LTD).*

By using the foregoing screener and the screening method, it is possible to reduce the dust particles, which are particles less than 10 $\mu$m in particle diameter, in the water-absorbent resin resulted from the drying process. The dust amount is preferably 0mg/m$^3$ to 500mg/m$^3$, more preferably 0mg/m$^3$ to 350mg/m$^3$. If the dust amount is more than 500mg/m$^3$, the dust will be scattered around in handling the water-absorbent resin, which needs to be absorbed in some manner.

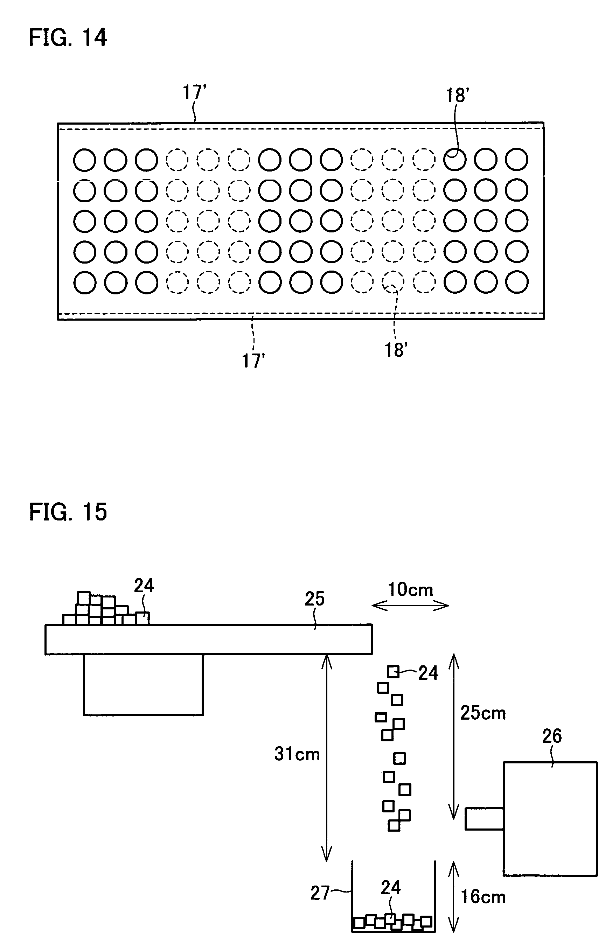

The foregoing screening device 300 is a rotation screener, but the screening device used in the present embodiment is not limited to this type of screener. For example, a vibration planer screening device may be used. Figure 13 shows a structure of a vibration planar screening device 600. The vibration planar screening device 600 has a planar first screening section 19 and a planar second screening section 17'. The second screening section 17' is made up of at least two layers which are opposed to each other. The first screening section 19 is provided beneath a plurality of second screening sections 17'. The net of the first screening section 19 and the punching 18' of the second screening section 17' have the same diameters as those of the screening device 300. Also in this planar screening device, as shown in Figure 14, the respective punchings 18' (shown by solid and broken lines) in the two layers of second screening section 17' are not opposed to each other.

The second screening section 17' of the vibration planar screening device 600 includes a product inlet 37 for supplying a water-absorbent resin and an excessively-large particle outlet 38. The path extending from the product inlet 37 is inclined downward as it approaches to the excessively-large particle outlet 38. The vibration planar screening device 600 has a vibration motor (not shown) to move the water-absorbent resin into the product inlet 37 and further move it to the second screening section 17'. The water-absorbent resin further travels to the punching where the excessively-small-diameter particles are removed by the first screening section 19. The excessively-small particles are then discharged from the excessively-large particle outlet 38. After that, the resin particles having been screened by the first screening section 19 are discharged from a product outlet 40. The long-sized particles which did not pass through the punching are discharged from the outlet 38.

Examples of vibration planar screening device include PS-280, PSL-300, PSL-400, and PSLL-400 (pellet screener; TANAKA IRON WORKS CO., LTD).

If a foreign body (a foreign body may be a metal piece which is the same in size and shape as the water-absorbent resin but different in color, garbage and/or water-absorbent resin with a small foreign body, a colored water-absorbent resin etc.) is mixed with the water-absorbent resin, the display effect of the water-absorbent resin decreases and also, if the foreign body is a metal piece, the degradation of the water-absorbent resin will be facilitated. It may also reduce the conservation stability of the water-absorbent resin. The foreign bodies are therefore preferably removed.

Such foreign bodies may be removed manually after found by human eye. Other way can be a method using a foreign body screener or a metal remover.

The metal remover may be either an electromagnet or a permanent magnet with a Gauss of 2000 to 12000, more preferably 5000 to 12000, further preferably 8000 to 12000.

(6. Plant-raising water retaining agent)

A Plant-raising water retaining agent according to the present invention contains the water-absorbent resin of the present invention. More specifically, the plant-raising water retaining agent contains the water-absorbent resin produced by the method for manufacturing water-absorbent resin according to the present invention. Since the water-absorbent resin contains less amount of residual monomer decreasing the physicality of the water-absorbent resin, it ensures superior hygiene and causes less odor. Owning to this advantage of the water-absorbent resin, the plant-raising water retaining agent according to the present invention is broadly free from problems of odor, insanitation or inadequate effect in raising plant, and capable of capturing a sufficient amount of water. Therefore, as long as it contains a certain amount of water, the water retaining agent constantly supplies water to plant. On this account, the plant is not required to be frequently watered.

The following more specifically explains the plant-raising water retaining agent. As long as it contains the water-absorbent resin according to the present invention, the plant-raising water retaining agent is not limited for its composition. However, a multivalent metal compound is preferably used.

The multivalent metal compound denotes salt or hydroxide of the multivalent metal, including bivalent metal such as calcium, magnesium, barium, alkali earth metal; and trivalent metal such as aluminum; or halide, normal/double inorganic salt such as nitrate, hydrosulfate, carbonate, organic salt such as lactic acid or fatty acid, or hydroxide or oxide of transition metals such as zinc, iron, manganese, copper or molybdenum.

In terms of stability of gel having been absorbed liquid, or provision of desirable environment for the physiological action to plants such as germination or growth, hydrosulfate, carbonate, nitrate, chloride, hydroxide, or oxide of calcium, aluminum, magnesium or zinc is preferable. Bivalent metal compound is more preferable. Calcium compound is further preferable.

The calcium compound is not particularly limited.

Examples of calcium compound include organic acid calcium such as calcium sulfate, calcium carbonate, calcium hydroxide, calcium oxide, calcium nitrate, calcium chloride, tricalcium phosphate, calcium borate, calcium lactate, calcium citrate, calcium stearate. Among them, calcium sulfate, calcium carbonate, calcium hydroxide, and calcium oxide are particularly preferable.

Further, the multivalent metal compound is completely or partially soluble to water. The solubility of the multivalent metal compound with respect to 100g of ion exchange water at 20°C is not more than 10.0g, preferably not less than 1.0g, more preferably not more than 0.5g, further preferably more than 0 but not more than 0.3g. When the solubility of the multivalent metal salt is higher than the foregoing range in the plant-raising water retaining material according to the present invention, the multivalent metal ion quickly infiltrates into the water-absorbent resin particles particularly in the case where water-absorbent resin contains a carboxyl group. As a result, the salt is exchanged with the salt of monovalent counterion (eg. sodium) in the water-absorbent resin, inducing internal metal crosslinking. Consequently, the water retaining property of the water retaining agent, such as speed or ratio of absorbency, greatly decreases. A water insoluble multivalent metal salt is not preferable, as it neither adhere nor bond to the surface of the water-absorbent resin, and does not infiltrate into the vicinity of the surface.

The multivalent metal compound is generally produced as particles containing crystallization water whose moisture content is 10 or less mass %. The diameter of the particle is not particularly limited, but is generally smaller than the mass average particle diameter of the water-absorbent resin. The amount of the multivalent metal and a specific method of adding the multivalent metal to the resin particle are specifically described later.

The plant-raising water retaining agent of the present invention is not particularly limited, but typically manufactured by the following three methods.

(1) A method of mixing multivalent metal compound slurry at least 50 mass % dense with water-absorbent resin particles containing 50 mass % or lower of moisture

(2) A method of mixing multivalent metal compound powder with water-absorbent resin particles containing 20 mass % or lower, more preferably 10 mass % or lower, of moisture, and adding aqueous liquid or water vapor into the product

(3) A method of mixing multivalent metal compound powder with water-absorbent resin particles containing 20 to 50 mass % of moisture

Apart from the methods (1) through (3), the following dry blending method may also be used. Multivalent metal compound powder is mixed with water-absorbent resin particles (A) containing, for example, 20 mass % or lower of moisture. However, because the multivalent metal compound is preferably adhered to the surface of the water-absorbent resin (A) or coating the surface of the water-absorbent resin, the methods (1) through (3) are more preferable.

Further, if the multivalent metal compound is mixed with the water-absorbent resin (A) in the form of solution or slurry containing more than 50 mass % of moisture, the multivalent metal compound is infiltrated into the water-absorbent resin. As a result, the chloride crosslinking occurs inside of the water-absorbent resin by the multivalent metal compound, which decreases the water-absorbent property of the plant-raising water retaining agent. This unwanted chloride crosslinking inside of the water-absorbent resin by the multivalent metal compound also occurs when the multivalent metal compound is added into the monomer in the polymerization process.

In the case of producing the plant-raising water retaining agent of the present invention through the method (1), the water-absorbent resin preferably contains low amount of moisture in consideration of prevention of the infiltration of the multivalent metal compound into the water-absorbent resin; namely 0 to 50 mass %, more preferably 0 to 40 mass %, further preferably 0 to 35 mass %, still further preferably 0 to 30 mass %, yet further preferably 0 to 20 mass %, most preferably 0 to 10 mass %.

Further, the multivalent metal compound is added to the water-absorbent resin in the form of solution or slurry.

Since the multivalent metal compound generally has low solubility, it is mixed with solvent such as aqueous liquid and then the resulting slurry is added to the water-absorbent resin. The density of multivalent metal compound of the slurry is preferably not less than 50 mass %, more preferably 50 to 90 mass %, further preferably 50 to 80 mass %. If the density is more than 90 mass %, the slurry looses fluidity, and becomes more like wet powder. The addition of compound thus becomes difficult. The amount of the slurry may vary depending on, for example, the type of multivalent metal compound. However, when aqueous liquid is used as a solvent of slurry, addition of large amount of slurry increases the moisture content of the water-absorbent resin. Therefore, the amount is preferably 0 to 50 mass %, more preferably 0 to 40 mass %, further preferably 0 to 30 mass %, still further preferably 0 to 20 mass %, yet further preferably 0 to 10 mass %, most preferably 0 to 5 mass %.The solvent used for the slurry containing the multivalent metal compound is not particularly limited as long as it allows the multivalent metal compound to be evenly dispersed. However, in order to cause the multivalent metal compound to be held to the surface of the water-absorbent resin or to be adhered to the surface of the water-absorbent resin, polar solvent, particularly water, is preferable. Further, an organic or inorganic dispersion agent may be added to adjust the fluidity of the slurry.

There are various ways of mixing the slurry with the water-absorbent resin, but the slurry is preferably sprayed into the water-absorbent resin or dropped into the water-absorbent resin. The slurry may be heated or cooled in consideration of temperature, solubility to water, density and fluidity, which all differ depending on the type of multivalent metal compound. In the case of heating the slurry, the temperature ranges from just above the congeal point to the boiling point, preferably 20 to 80 °C.

In the case of producing the plant-raising water retaining agent of the present invention through the method (2), multivalent metal compound powder is mixed with water-absorbent resin particles containing 20 mass % or lower, more preferably 10 mass % or lower, of moisture, and aqueous liquid or water vapor is then added into the product. In the case of adding aqueous liquid, the foregoing aqueous liquid (1) is used. The amount is preferably 0 to 30 mass %, more preferably 0 to 20 mass %, further preferably 0 to 15 mass %, still further preferably 0 to 10 mass %, most preferably 0 to 5 mass %.

(7. Artificial ice for display)

A display-use artificial ice according to the present invention contains the water-absorbent resin of the present invention. The other components of the artificial ice are not particularly limited, but one example is coloring agent. The water-absorbent resin according to the present invention is mainly constituted of particles of polyhedron shape mainly constituted of 6-plane particles.

The particle size of water-absorbent resin of the present invention is larger than that of general water-absorbent resin (mass average particle diameter = approximately 400μm). The water-absorbent resin of the present invention has superior water capturing property. The appearance of the artificial ice is therefore similar to that of real ice. With this advantage the artificial ice is suitable for artificial food for display purposes generally used in a restraint.

The present invention is not limited to the description of the embodiments above, but may be altered by a skilled person within the scope of the claims. An embodiment based on a proper combination of technical means disclosed in different embodiments is encompassed in the technical scope of the present invention. [Examples]

The present invention is more specifically explained below based on concrete examples. The present invention is however not limited to the examples. The present invention allows a person skilled in the art to perform various modifications in the scope of the present invention. The various parameters were measured by the following methods.

(1) Absorbency

The absorbency designates an absorption rate of water-absorbent resin with respect to 0.90 mass % physiological saline. The absorbency is measured after 48 hours under no pressure. A process is shown below.

0.200g of water absorbent resin was evenly poured into a nonwoven fabric bag (Nangoku Pulp Co. Ltd.; Heatron Paper GSP-22 (85mm × 60mm)) and was thermally sealed. Then the bag was soaked in a far extra amount (typically 500g) of 0.90 mass % sodium chloride aqueous solution (physiological saline) at room temperature.

The bag was drawn from the solution after 48 hours, and was dried for three minutes by a centrifugal machine (Centrifugal machine H-122, Kokusan Co. Ltd.) with a centrifugal force (250G) according to "edana ABSORBENCY II 441, 1-99". After that, a mass W2 (g) of the bag was measured. The same experiment was performed without water-absorbent resin or water-absorbent composition, and a mass W1 (g) was measured. Based on W1 and W2, the absorbency was found as follows (g/g).

$$\text{Absorbency (g/g)} = (\text{mass W2 (g)} - \text{mass w1 (g)})/(\text{mass (g) of water-absorbent resin} - 1) \quad \cdots \text{(1)}$$

(2) Residual monomer

The content of residual monomer in the water-absorbent resin is measured by one of publicly-known conventional methods. In one example method, 1.0g of water-absorbent resin is added to 184.3g of 0.90 mass % sodium chloride aqueous solution (physiological saline), and the mixture is stirred for two hours for extraction. As a result, the water-absorbent resin becomes a swollen gel, which is filtered by a filter paper. The amount of residual monomer in the filtrate is then analyzed by liquid chromatography. In this case, an analytical curve obtained in the same manner as that for finding monomer standard solution of a known density is used as an external standard, and the amount of residual monomer in the water-absorbent resin is found by taking dilution ratio of the filtrate into account.

(3) Measurements of mass average particle diameter and logarithm standard deviation of hydrous gelatinous polymer

A 30g sample of hydrous gelatinous polymer was added to 1000g of 20 mass % NaCl aqueous solution, and the mixture was stirred for 120 minutes by rotating a stirrer chip at 300 rpm. After the stirring, a 100g sample was taken together with 6000g of 20 mass % NaCl solution into 5-8 kinds of JIS standard sieves Z8801-1 (1998) (The IIDA TESTING SIEVE; internal diameter: 20 cm) respectively having mesh sizes of 16.0mm, 13.2mm, 11.2 mm, 9.5 mm, 8.0 mm, 6.70 mm, 5.6 mm, 4.75 mm, 4.0 mm, 3.35 mm, 2.8 mm, 2.36 mm, 2.0mm, and 1.0 mm for classification. The sieves are capable of measuring the specific logarithm standard deviation (R=15.9%, 50.0%, 84.9% in Formula (2)) The sample on each sieve was sufficiently drained and was measured by a scale.

The particle size distribution of the particulate hydrous gelatinous polymer was plotted on a logarithm probability paper based on the following formula (2) where w expresses mass of particulate hydrous gelatinous polymer after the classification and draining, r expresses the mesh size, and w0 expresses the mass of sample, which is 30g. The average particle diameter of the particulate hydrous gelatinous polymer was determined by finding a particle diameter at a time where R (gross sieve %) becomes 50 mass %.

$$R\ (\alpha)\ =\ (w0/w)^{1/3}\times r\ \ldots\ (2)$$

The logarithm standard deviation σζ was found by calculating respective particle diameters (X1, X2) at the times where R (gross sieve %) = 84.1% and R = 15.9%. Then the logarithm standard deviation σζ was found according to the following formula (3).

$$\sigma\zeta\ =\ (1/2)\ \ln\ (X2/X1)\cdots(3)$$

(4) Measurements of mass average particle diameter and logarithm standard deviation of water-absorbent resin

A 100g sample of water-absorbent resin was taken into 5-8 kinds of JIS standard sieves Z8801-1(1998) capable of measuring a logarithm standard deviation (R=15.9%, 50.0%, 84.9% in Formula (2)) among plural kinds of the IIDA TESTING SIEVE (internal diameter: 20 cm) respectively having mesh sizes of 16.0mm, 13.2mm, 11.2 mm, 9.5 mm, 8.0 mm, 6.70 mm, 5.6 mm, 4.75 mm, 4.0 mm, 3.35 mm, 2.8 mm, 2.36 mm, 2.0mm, and 1.0 mm.

Then the classification was performed by a vibration classifying device (IIDA SIEVE SHAKER ES-65 Serial No. 0501) for five minutes. The sample on each sieve was measured by a scale. The particle size distribution of the water-absorbent resin was plotted on a logarithm probability paper based on the foregoing formula (2) where w expresses mass of particulate hydrous gelatinous polymer after the classification and draining, r expresses the mesh size, and w0 expresses the mass of sample, which is 30g. The average particle diameter of the water-absorbent resin was determined by finding a particle diameter at a time where R (gross sieve %) becomes 50 mass %.

The logarithm standard deviation σζ was found by calculating respective particle diameters (X1, X2) at the times where R (gross sieve %) = 84.1% and R = 15.9%. Then the logarithm standard deviation σζ was found based on X1 and X2 according to the foregoing formula (3).

(5) Light resistance test

Ion exchange water in an amount of 25 times the solid content of water-absorbent was completely absorbed to the resin, and a resulting gel (30g) was poured into a 225cc mayonnaise bottle. After the lid is placed, the bottle was set outside for a week, before the result was observed visually.

(6) Measurement of shape (faceplate) of hydrous gel

A 30g sample of hydrous gelatinous polymer resulted from the cutting process was taken, and the shape (faceplate) of each piece was observed visually so as to find a mass percentage of the hydrous gelatinous polymer with 4-12 planes or a mass percentage with 6 planes.

Concentration (mass %) of a hydrous gelatinous polymer with 4-12 planes (or 6 planes) = mass (g) of the hydrous gelatinous polymer with 4-12 planes (or 6 planes / 30 (g) × 100

(7) Measurement of moisture content of hydrous gelatinous polymer (Solid content)

A part of the hydrous gelatinous polymer taken from the polymerizer was cut out and immediately cooled, and was quickly cut into pieces. 4g of the resulting pieces of the hydrous gelatinous polymer was taken into a petri dish 50mm in internal diameter, and was dried by being placed still in a 180°C stationary dryer for 16 hours.

$$\text{Moisture content (mass \%) of a hydrous gelatinous polymer} = 100 - \left(\text{mass (g) of the dried hydrous gelatinous polymer / mass (g) of the hydrous gelatinous polymer before dried} \times 100\right)$$

(8) Measurement of moisture content of water-absorbent resin (Solid content)

2g of water-absorbent resin was taken into a petri dish 50mm in internal diameter, and was dried by being placed still in a 180°C stationary dryer for 16 hours.

$$\text{Moisture content (mass \%) of a water-absorbent resin} = 100 - \left(\text{mass (g) of the dried water-absorbent resin / mass (g) of the water-absorbent resin before dried} \times 100\right)$$

Note that, the solid content can be found by inverse calculation as follows according to the moisture content.

$$\text{Solid content (mass \%)} = 100 - \text{moisture content (mass \%)}$$

(9) Measurement of dust amount

A vibration feeder 25 (CF-2; Sinko electronics Co. Ltd.), a digital dust scale 26 (P-5L; Shibata Kagaku Kikai Co. Ltd.), and a receiving cup 27 are placed at certain intervals as shown in Figure 15.

First, 1 kg of the water-absorbent resin 24 is placed on the vibration feeder 25, and the vibration feeder is adjusted so that the resin will land on the cup after a minute after departing the feeder 25. After that, 1kg of the water-absorbent resin 24 is placed again on the vibration feeder 25, and the resin is released and the digital dust scale is started to run for a minute simultaneously. After a minute, the dust amount is found according to the value of the digital dust scale.

[Example 1]

A monomer aqueous solution containing 254.50g of 37 mass % sodium acrylate aqueous solution, 29.50g of acrylic acid, 0.747g of 10 mass % polyethyleneglycol diacrylate (n=9) aqueous solution, 20.26g of ion exchange water, 6.25g of 10 mass % polyethyleneglycol (Polyethylene Glycol 6000: product of Kanto Kagaku Co. Ltd.) aqueous solution was prepared. In other words, an ethylene unsaturated monomer aqueous solution including 100 mole% (excluding crosslinking agent) of acrylic acid and sodium acrylate was prepared. Nitrogen was added to the monomer aqueous solution until the dissolved oxygen density becomes 0.1ppm or lower.

Then, 1.41g of 1.0 mass % hydroxycyclohexylphenylketone (Irgacure 184; product of Chiba specialty chemical Co. Ltd.) acrylic acid solution and 1.41g of 3.0 mass % sodium persulfate aqueous solution were poured into a 30cm × 30cm tray (product of PFA) in this order, and then a monomer solution was added. The mixture was subjected to polymerization with a 450W high-pressure mercury lamp (Toscure 401; product of Harison Toshiba lighting Corp.). The light irradiation distance was 60cm.

The polymerization was started at 22°C, and the temperature increased to 80°C after 2 and a half minutes. After five minutes light irradiation with the high-pressure mercury lamp, the lamp was turned off to stop polymerization.

The moisture content of the hydrous gel was 55 mass % at this stage.

The polymerization sheet was 5 mm in thickness. The obtained hydrous gel sheet was cut by scissors into 5mm blocks so as to produce an angular hydrous gel with 6 smooth planes (1). The amount of hydrous gel with 6 planes was 98.5 mass %. Further, the mass average particle diameter of the hydrous gelatinous polymer was 5.0mm and logarithm standard deviation of particle size distribution was 0.20. 300g of the hydrous gel (1) was poured into a 5L M5R-type Lödige mixer (MATSUBO Corporation.). While dropping 0.75g (0.05 mass %) of 10 mass % aluminum sulfate aqueous solution and 0.03g (0.02 mass %) of 10 mass % Diethylene Triamine Pentaacetic Acid (Chelest PC45, Chelest Co. Ltd.) aqueous solution into the mixer, the hydro gel was stirred at 200min$^{-1}$ for 1 minute by a syringe to be mixed with the solutions. The resulting hydrous gel grains did not aggregate.

The hydrous gel was spread on a 50 metal gauze (mesh size = 300 $\mu$m), and dried for 30 minutes at 130 °C. Obtained was a water-absorbent resin (1) 20 mass % in moisture content. The average particle diameter and the logarithm standard deviation of this water-absorbent resin were 3.8mm and 0.19, respectively. Further, absorbency (hereinafter referred to as CRC) was 41.0g/g, and the content of residual monomer was 100ppm. No change in color or shape was observed in the light resistance test.

The measurements of average particle diameter and the logarithm standard deviation for the hydrous gelatinous polymer and the water-absorbent resin were carried out by using sieves of 8.0mm, 6.70mm, 5.6mm, 4.75mm, 4.0mm, 2.8mm, and 2.0mm.

[Example 2]

A monomer aqueous solution containing 407.2g of 37 mass % sodium acrylate aqueous solution, 47.2g of acrylic acid, 1.195g of 10 mass % polyethyleneglycol diacrylate (n=9) aqueous solution, 40.35g of ion exchange water, 10.00g of 10 mass % polyethyleneglycol (Polyethylene Glycol 6000: product of Kanto Kagaku Co. Ltd.) aqueous solution was prepared. In other words, an ethylene unsaturated monomer aqueous solution including 100 mole% (excluding crosslinking agent) of acrylic acid and sodium acrylate was prepared. Nitrogen was added to the monomer aqueous solution until the dissolved oxygen density becomes 0.1ppm or lower.

Then, 2.256g of 1.0 mass % hydroxycyclohexylphenylketone (Irgacure 184; product of Chiba specialty chemical Co. Ltd.) acrylic acid solution and 2.256g of 3.0 mass % sodium persulfate aqueous solution were poured into a 30cm × 30cm tray (product of PFA) in this order, and then a monomer solution was added. The mixture was subjected to polymerization with a 450W high-pressure mercury lamp (Toscure 401; product of Harison Toshiba lighting). The light irradiation distance was 60cm. The polymerization was started at 22°C, and the temperature increased to 88°C after 4 minutes. The polymerization sheet was 8 mm in thickness.

The obtained hydrous gelatinous sheet was cut by scissors into 8mm blocks so as to produce an angular hydrous gel with 6 smooth planes (2). The amount of hydrous gel with 6 planes was 97.0 mass %. Further, the mass average particle diameter of the hydrous gelatinous polymer was 8.0mm and logarithm standard deviation of particle size distribution was 0.18. 300g of the hydrous gel (2) was poured into a 5L M5R-type Lödige mixer (MATSUBO Corporation.). While dropping 0.75g (0.05 mass %) of 10 mass % aluminum sulfate aqueous solution and 0.105g (0.05 mass %) of 2-hydroxybenzophenone-4-diglycerylether into the mixer, the hydro gel was stirred at 200min$^{-1}$ for 1 minute by a syringe to be mixed with the solutions. The resulting hydrous gel grains did not aggregate.

The hydrous gel was spread on a 50 metal gauze (mesh size = 300 $\mu$m), and dried for 30 minutes at 130 °C. Obtained was a water-absorbent resin (2) 19 mass % in moisture content. The average particle diameter and the logarithm standard deviation $\sigma\zeta$ of this water-absorbent resin were 6.8mm and 0.17, respectively. Further, absorbency (hereinafter referred to as CRC) was 41.0g/g, and the content of residual monomer was 80ppm. No change in color or shape was observed in the light resistance test.

The measurements of average particle diameter and the logarithm standard deviation for the hydrous gelatinous polymer and the water-absorbent resin were carried out by using sieves of 11.2mm, 9.5mm, 8.0mm, 6.70mm, 5.6mm, 4.75mm, 4.0mm, and 2.8mm.

[Example 3]

An aqueous solution was prepared by sufficiently mixing monomers of 43.7kg of acrylic acid and 286kg of 37 mass % sodium acrylate aqueous solution, 337g of polyethyleneglycol diacrylate (n=9), and 86.3g of ion exchange water. In other words, an ethylene unsaturated monomer aqueous solution including 100 mole% (excluding crosslinking agent) of acrylic acid and sodium acrylate was prepared. A fluorinated adhesion tape containing glass fiber (TGF Glass Cloth; product of Yodogawa Hu-Tech) was bonded onto an endless steal belt, and a belt polymerizer driven at 1.0m/min was placed on the tape. The belt polymerizer has plural caulds 3.0cm in height at 30 cm intervals. The aqueous solution was supplied to the belt polymerizer at 1.2kg/min, so that the aqueous solution became 5mm high. The aqueous solution was heated on the supplying path so that it becomes 22°C at the entrance of the belt polymerizer. Further, Nitrogen was added to the monomer aqueous solution on the supplying path until the dissolved oxygen density becomes 0.5ppm or lower.

An acrylic acid solution of 0.01g hydroxycyclohexylphenylketone (Irgacure 184; product of Chiba specialty chemical Co. Ltd.) and an aqueous solution of 0.03g of sodium persulfate were alternatively injected between the monomer aqueous solutions reduced in dissolved oxygen density. The monomers were then sufficiently mixed with these polymerization initiators (monomer density = 40.0 mass %).

The mixture was then irradiated with UV for 4 minutes with 6 high-pressure mercury lamps placed at 3m intervals at 20cm high from the liquid surface of aqueous solutions. The aqueous liquid supplied to the belt polymerizer reached 90 °C after 3 and a half minutes, which is a peak temperature of polymerization. Nitrogen was added to the monomer aqueous solutions at 5L/min simultaneously from two sources: the same position as the supplying entrance, and the center of the belt.

The moisture content of the resulting gel was 55.4 mass %, and the thickness was 4 mm. The hydrous gel sheet came out from the polymerizer was supplied into the cutting device 100 (Figure 3) at a speed of 1.0m/min. The gel sheet was then cut into blocks (hydrous gel (3)). The hydrous gel (3) was mainly constituted of particles with 6 smooth planes, specifically 85 mass %. The mass average particle diameter and the logarithm standard deviation of the hydrous (3) gel were 4.0 mm and 0.20, respectively.

At that stage, propyleneglycol was sprayed at 8g/min from the respective 2-fluid nozzles (BIMV11002: product of H.Ikeuchi&Co., Ltd.) of the spraying devices 9, 10 and 11 under 0.1 MPa of air pressure. Further, propyleneglycol aqueous solution was sprayed from a gear pump of the spraying device 12 at 10g/min. The whole amount of propyleneglycol was 7.08 wt% with respect to the solid content of water-absorbent resin.

5000g of the hydrous gel (3) was poured into a 20L Lödige mixer (M20-type; MATSUBO Corporation.). While dropping 22.3g (0.10 mass %) of 10 mass % aluminum sulfate aqueous solution into the mixer, the hydro gel was stirred at 200min-1 for 1 minute by a syringe to be mixed with the solution. The resulting hydrous gel grains did not aggregate.

The hydrous gel was spread on a 50 metal gauze (mesh size = 300 $\mu$m), and dried for 30 minutes at 130 °C. Obtained was a water-absorbent resin (3) 20 mass % in moisture content. The average particle diameter and the logarithm standard deviation $\sigma\zeta$ of this water-absorbent resin (3) were 3.5mm and 0.18, respectively. Further, absorbency (hereinafter referred to as CRC) was 42.0g/g, and the content of residual monomer was 120ppm.

The measurements of average particle diameter and the logarithm standard deviation for the hydrous gelatinous polymer and the water-absorbent resin were carried out by using sieves of 8.0mm, 6.70mm, 5.6mm, 4.75mm, 4.0mm, 2.8mm and 2.0mm.

[Example 4]

The process was the same as Example 3 except that 50 mass % propyleneglycol aqueous solution was used in the cutting device 100 instead of the adhesion block agent.

The hydrous gel grains resulted from the cutting process did not aggregate. The hydrous gel particles contain 6-plane particles at a ratio of 80 mass%. The mass average particle diameter and the logarithm standard deviation of this hydrous gel were 4.2mm and 0.18, respectively.

This hydrous gel was spread on a 50 metal gauze (mesh size = 300 $\mu$m), and dried for 30 minutes at 130 °C. Obtained was a water-absorbent resin (4) 19 mass % in moisture content. The average particle diameter and the logarithm standard deviation of this water-absorbent resin (4) were 3.7mm and 0.17, respectively. Further, the CRC was 42.0g/g, and the content of residual monomer was 120ppm.

[Example 5]

2kg of hydrous gel sheet obtained by the process of Example 3 was dried as such for 30 minutes at 120°C. The moisture content of the dried sheet gel was 39.3%.

The dried sheet gel was cut into blocks in the same manner as that of Example 3. The resulting hydrous gel grains did not aggregate. The content of 6-plane particles were 95 mass %. The average particle diameter and the logarithm standard deviation of this hydrous gel were 5.0mm and 0.18, respectively.

This hydrous gel was spread on a 50 metal gauze (mesh size = 300 $\mu$m), and dried for 30 minutes at 120 °C. Obtained was a water-absorbent resin (5) 20 mass % in moisture content. The average particle diameter and the logarithm standard deviation of this water-absorbent resin (5) were 4.5mm and 0.18, respectively. Further, the CRC was 43.0g/g, and the content of residual monomer was 115ppm.The measurements of average particle diameter and the logarithm standard deviation for the hydrous gelatinous polymer and the water-absorbent resin were carried out by using sieves of 8.0mm, 6.70mm, 5.6mm, 4.75mm, 4.0mm, 2.8mm and 2.0mm.

[Example 6]

300g of the water-absorbent resin (3) obtained by the method of Example 3 was poured into a 5L Lödige mixer (M5R-type; MATSUBO Corporation.). While dropping 60g of 50 mass % dense slurry of calcium sulfate into the mixer, the hydro gel was stirred at 330rpm for 15 seconds to be mixed with the slurry. The mixture was then dried for 10 minutes by hot air of 120 °C. The plant-raising water-retaining agent (1) was thus obtained.

150ml of ion exchange water was poured into a polypropylene container 80 mm in internal diameter and 250 mm in height. 1.5g of the plant-raising water-retaining agent (1) was poured into the container and the container was placed still for an hour. In this way the plant-raising water-retaining agent was processed into a gel culture medium (absorbency × 100). 30 radish sprouts seeds were sowed in the medium and a nonwoven fabric lid was placed. The radish sprout was cultured for a week in a cultivation room (25 °C, 500Lux, day length = 12 hours) before germination.

After a week, the developed shoot of radish sprout was taken out from the room. The length of stem (length of stem from the ground), which was measured from the base portion with the shoot to the end of the leaf, was 610 mm, and the length in the soil (depth of stem under the ground), which is measured from the base portion with the shoot to the end of the main root, was 560 mm. The root hair was observed.

[Example 7]

Ion exchange water was absorbed into the water-absorbent resin (3) produced by the method of Example (3) so that the resin was swollen into a size of 100 times large. The artificial ice for display purpose was thus produced.

[Example 8]

An aqueous solution was prepared by sufficiently mixing monomers of 15.9kg of acrylic acid and 173.4kg of 37 mass % sodium acrylate aqueous solution, 104.6g of polyethyleneglycol diacrylate (n=9), 7.9g of ion exchange water, and 2.7g of 15 mass % of polyethylene glycol (product name: polyethylene glycol 6000 Nippon Oil Corporation). The aqueous solution was adjusted to 20°C. Nitrogen gas was added to the monomer aqueous solution and the content of dissolved oxygen was adjusted to 1ppm. Then the disappearance of bubbles was visually confirmed.

Next, 3 mass % of solvent containing acrylic acid was prepared by dissolving 0.01g hydroxycyclohexylphenylketone (Irgacure 184; product of Chiba specialty chemical Co. Ltd.) in a solution in which acrylic acid and water are mixed at a ratio of 1:1 (mass ratio). Next, 3 mass % of sodium persulfate aqueous solution was prepared.

A fluorinated adhesion tape (product name: CHEMLAM6R Saint-Gobain KK.) containing glass fiber was bonded onto an endless steal belt, and caulds 1.0 cm in height are placed thereon at 22 cm intervals. The monomer aqueous solution was supplied to a belt polymerizer driven at 1.0m/min at 1.4kg/min, the hydroxycyclohexylphenylketone acrylic acid and water mixture solution (mass ratio = 1:1) was supplied to the belt polymerizer at 10g/min, and the sodium persulfate aqueous solution was supplied to the belt polymerizer at 10g/min.

The mixture was then irradiated with UV for 4.3 minutes with a mercury lamp (Toshiba MERCURY LAMP H400BL-L). The gross light quantity was 820mJ/cm$^2$. Polymerization was carried out by supplying nitrogen gas at 50L/min.

The moisture content of the resulting gel was 45.0 mass %, and the thickness was 4.5 mm. The maximum temperature in the polymerization was 90 °C. No bubbles greater than 200$\mu$m were visually seen in the hydrous gel sheet. The hydrous gel sheet was supplied from the polymerizer to the cutting device 100 (shown in Fig. 1) via a roller conveyer horizontally placed at a speed of 1.0m/min. Then the hydrous gel sheet was cut into pieces 4mm in width and 4mm in length. As a result, hydrous gel angular particles (4) each having 6 smooth planes are obtained. As shown in Figure 1b, the scrapers 3 and 4 of the cutting device 100 were partially coated with fluorine resin enamel paint (product name: 420-105 ONE COAT GRAY Du-Pont Co. Ltd.), more specifically, each plane of the scrapers 3 and 4 through which the hydrous gel sheet which had been cut in the width direction by the slitter rolls 1 and 2, which are vertical-type cutter, passed was coated with the fluorine resin enamel paint. With this arrangement, no blockage occurs in the cutting device 100.

A cool air of 25 °C was applied to the hydrous gel sheet from the upper part of the roller conveyer, and therefore the surface of the gel sheet had a temperature of 50 °C immediately before supplied into the cutting device 100. The content of the 6-plane hydrous gel particles was 99 mass %. The mass average particle diameter and the logarithm standard deviation of the hydrous gel (4) were 4.4 mm and 0.19, respectively.

In the cutting process, propyleneglycol was sprayed at 30g/min from the respective spraying nozzles of the spraying devices 9, 10 and 11 under 0.1MPa of air pressure. Further, propyleneglycol aqueous solution was sprayed from a gear pump of the spraying device 12 at 30g/min. The whole amount of propyleneglycol was 19.05 mass% with respect to the solid content of water-absorbent resin.

40kg of the hydrous gel (4) was poured into a 130L batch-type Lödige mixer (MATSUBO Corporation). The hydrous gel (4) was stirred at a blade speed of 140min - 1, and at a side chopper speed of 3000min - 1. While spraying 600g of 10 mass % aluminum sulfate aqueous solution from a spraying nozzle into the mixer, the hydro gel was stirred. The resulting hydrous gel particles were not aggregate.

The hydrous gel was spread on a 50 metal gauze (mesh size = 300 $\mu$m) of a batch-type hot air drier, and was dried for 30 minutes at 105 °C with an airflow of 1m/s. The temperature of the hydrous gel (4) increased to 105°C after 30 minutes. Then the hydrous gel (4) was further dried for 50 minutes at 150 °C with an airflow of 1m/s. The temperature of the hydrous gel (4) increased to 149°C after 50 minutes. Obtained was a water-absorbent resin (6) 12 mass % in moisture content.

The water-absorbent resin (6) was then supplied to a screener 300 at a speed of 1.5kg/min. Obtained was a water-absorbent resin (7). Note that, the first screening section 19 of the screener 300 was provided with a 2.8mm net, and dry air was sent to the resin from a portion below the first screening section 19 at 200L/min. The second screening section 17 had a double-layered structure constituted of an internal screening section with 7mm punchings and an outer screening section with 9mm punchings. The gap between the respective punchings was 7mm.

The average particle diameter and the logarithm standard deviation $\sigma\zeta$ of this water-absorbent resin (7) were 4.0mm and 0.18, respectively. Further, CRC was 37.0g/g, and the content of residual monomer was 280ppm. The dust amount was 220mg/m$^3$. The content of the 6-plane water-absorbent resin (7) was 99 mass %.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 9]

The same method as that of Example 8 was performed except that the drying of gel was carried out by a band drier. The hydrous gel (4) passed through 5 rooms identical in length, staying in each room for 120 minutes. The rooms 1 through 3 were kept at 105°C, the rooms 4 and 5 were kept at 150°C, and the air was sent at 1.0m/s. Obtained was a water-absorbent resin (8).

The temperature of the hydrous gel (4) which had Come out of the third room was 105°C. The temperature of the hydrous gel (4) which had come out of through the fifth room was 148°C. In the drying process, the hydrous gel (4) had a height of 4cm.

The average particle diameter and the logarithm standard deviation $\sigma\zeta$ of this water-absorbent resin (9) were 4.0mm and 0.18, respectively. Further, CRC was 38.0g/g, and the content of residual monomer was 290ppm. The dust amount was 250mg/m$^3$. The content of the 6-plane water-absorbent resin (9) was 98 mass %. No bubbles were visually seen inside the water-absorbent resin (9).

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 10]

The same method as that of Example 8 was performed except that the drying of gel was carried out by a rotary-type through flow drier (SRTA-2), which is a rotation-type ventilation drier provided by Okawara Seisakusho.

16kg of the hydrous gel (4) was supplied to the SRTA-2, and was rotated at 8rpm with hot air of 190°C at a wind speed of 1.0m/s. Each punching of the drum was 1.0mm in diameter. The temperature of the hydrous gel (4) increased to 110°C after 40 minutes, and further increased to 149°C after 80 minutes. The drying was stopped after 80 minutes. Obtained was a water-absorbent resin (10).

The water-absorbent resin (10) was screened by the screener 300. The moisture content of the resulting water-absorbent resin (11) was 13.8 mass %.

The average particle diameter and the logarithm standard deviation $\sigma\zeta$ of this water-absorbent resin (11) were 3.9mm and 0.17, respectively. Further, CRC was 42.1g/g, and the content of residual monomer was 270ppm. The dust amount was 190mg/m$^3$. The content of the 6-plane water-absorbent resin (11) was 98 mass %.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 11]

Five metal pieces were added to the water-absorbent resin (7) to produce a water-absorbent resin (12). The water-absorbent resin (12) was supplied to a metal remover at 1.5kg/min. The water-absorbent resin (12) was then visually examined. The five metal pieces were completely removed.

Note that, in the metal remover, the water-absorbent resin (12) passed through three stages of $3 \times 10000$ Gauss permanent magnets each 10cm in length placed at 2cm intervals.

[Example 12]

The first one of the black light mercury lamps (Toshiba MERCURY LAMP H400BL-L) used in Example 8 is replaced with an UV-152/1MNSC3-AA06 (product of Ushio Denki Co. Ltd.) equipped with a metal halide lamp (light-emission length = 125mm). The shutter was opened at 1.0kW. The gross light quantity was 1300mJ/cm$^2$. To a belt polymerizer driven at 1.5m/min, the same monomer aqueous solution as that of Example 8 was supplied at 2.1kg/min, the hydroxycyclohexylphenylketone acrylic acid and water mixture solution (mass ratio = 1:1) was supplied at 10g/min, and the sodium persulfate aqueous solution was supplied at 15g/min.

The moisture content of the resulting gel was 45.1 mass %, and the thickness was 4.5 mm. The maximum temperature in the polymerization was 94 °C. No bubbles greater than 200$\mu$m were visually seen in the hydrous gel sheet. The hydrous gel sheet was supplied from the polymerizer to the cutting device 100 (shown in Fig. 3) via a cooling belt horizontally placed at a speed of 1.5m/min. Then the hydrous gel sheet was cut into pieces 4mm in width and 4mm in length. As a result, hydrous gel angular particles (7), which are all substantially angular and each has a 6 smooth plane, are obtained.

As shown in Figure 4, the scrapers 3 and 4 of the cutting device 100 were partially coated with fluorine resin enamel paint (product name: 420-105 ONE COAT GRAY Du-Pont Co. Ltd.), more specifically, each plane of the scrapers 3 and 4 through which the hydrous gel sheet which had been cut in the width direction by the vertical-cutting blades 1 and 2 passed was coated with the fluorine resin enamel paint. With this arrangement, no blockage occurs in the cutting device 100.

The cooling belt was chilled by cold water of 13 °C which is applied from the bottom, and therefore the surface of the gel sheet had a temperature of 40 °C immediately before supplied into the cutting device 100. The content of the 6-plane hydrous gel particles was 99 mass %. The mass average particle diameter and the logarithm standard deviation of the hydrous gel (5) were 4.3 mm and 0.19, respectively.

In the cutting process, propyleneglycol was sprayed at 65g/min from the respective spraying nozzles of the spraying devices 9', 10' and 11. Further, propyleneglycol aqueous solution was sprayed from a gear pump of the spraying device 12 at 65g/min. The whole amount of propyleneglycol was 27.45 mass% with respect to the solid content of water-absorbent resin.

60kg of the hydrous gel (7) was poured into a 130L batch-type Lödige mixer (MATSUBO Corporation). The hydrous gel (5) was stirred at a blade speed of 140min - 1, and at a side chopper speed of 3000min - 1. While spraying 900g of 10 mass % aluminum sulfate aqueous solution into the mixer, the hydro gel was further stirred. The resulting hydrous gel particles were not aggregate.

Then the hydrous gel passed through 5 rooms identical in length, staying in each room for 120 minutes.

The rooms 1 through 3 were kept at 105°C, the rooms 4 and 5 were kept at 150°C, and the air was sent at 1.0m/s in the form of up-blowing in the rooms 1 and 3, and in the form of down-blowing in the rooms 2, 4 and 5. The temperature of the hydrous gel (7) which had come out of the third room was 105°C. The temperature of the water-absorbent resin which had come out of through the fifth room was 148°C. In the drying process, the hydrous gel (4) had a height of 4cm. The hydrous gel (4) was screened by the same method as that described in Example (8). As a result the water-absorbent resin (13) was obtained.

The moisture content of the water-absorbent resin (13) was 12.5 mass %. The average particle diameter and the logarithm standard deviation $\sigma\zeta$ of this water-absorbent resin (7) were 4.4mm and 0.19, respectively. Further, CRC was 51.0g/g, and the content of residual monomer was 260ppm. The dust amount was 250mg/m$^3$. The content of the 6-plane water-absorbent resin (13) was 99 mass %.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 13]

An aqueous solution was prepared by sufficiently mixing monomers of 15.9kg of acrylic acid and 173.4kg of 37 mass % sodium acrylate aqueous solution, 104.6g of polyethyleneglycol diacrylate (n=9), and 7.9g of ion exchange water. The aqueous solution was adjusted to 20°C. The aqueous solution was transported on a supplying line at 1.75kg/min, and nitrogen gas was continuously sent to the monomer aqueous solution on the supplying belt at 200ml/min. After that, the bubbles were removed by using a defoaming device (QUICK TORON® 01-type: Nippon Oil Corporation) which has the same structure as that of the defoaming device 400 of Figure 1. The monomer aqueous solution having been thus deaerated was discharged from the outlet holes of QUICK TORON® at 1.4kg/min. To this solution, 15 mass % polyethyleneglycol (Polyethylene Glycol 6000: Nippon Yushi Co. Ltd.) aqueous solution was supplied at 18.9g/min, the same hydroxycyclohexylphenylketone acrylic acid and water mixture solution (mass ratio = 1:1) as that of Example 8 was supplied at 10g/min, and the same sodium persulfate aqueous solution as that of Example 8 was supplied at 10g/min.

The content of dissolved oxygen of the monomer aqueous solution was 5ppm. No bubbles were seen in the monomer aqueous solution. Meanwhile, the monomer aqueous solution containing bubbles was discharged at 0.35kg/min. The discharged solution was supplied back to the monomer producing tank.

The same processes after polymerization as those of Example 8 were performed with respect to this monomer aqueous solution. The moisture content of the resulting hydrous gel was 45.3 mass %. The thickness and the logarithm standard deviation $\sigma\zeta$ of this hydrous gel were 4.5mm and 0.19, respectively.

The hydrous gel particles were screened by a screener 300, and a water-absorbent resin (14) was obtained. The average particle diameter and the logarithm standard deviation $\sigma\zeta$ of this water-absorbent resin (14) were 4.0mm and 0.19, respectively. Further, CRC was 42.4g/g, and the content of residual monomer was 280ppm. The dust amount was 220mg/m$^3$.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 14]

The same monomer aqueous solution as that of Figure 8 was prepared except that the removal of dissolved oxygen from the solution having been adjusted to 20°C was performed by a deaeration module (MHF0504MBFT: MITSUBISHI RAYON ENGINEERING CO., LTD) which has the same structure as that of the defoaming device 500 of Figure 2. Deaeration was performed by a vacuum pump at 7kPa, and the amount of the dissolved oxygen became 1ppm. No bubbles were seen in the monomer aqueous solution. The same processes after polymerization as those of Example 8 were performed with respect to this monomer aqueous solution.

The moisture content of the resulting hydrous gel was 45.0 mass %. The average particle diameter and the logarithm standard deviation $\sigma\zeta$ of this hydrous gel were 4.5mm and 0.19, respectively.

The hydrous gel particles were screened by a screener 300, and a water-absorbent resin (15) was obtained. The average particle diameter and the logarithm standard deviation $\sigma\zeta$ of this water-absorbent resin (15) were 3.9mm and 0.19, respectively. Further, CRC was 42.0g/g, and the content of residual monomer was 275ppm. The dust amount was 260mg/m$^3$.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 15]

The same method as that of Example 8 was performed except that the hydrous gel was dried for 60 minutes at 150 °C with an airflow of 1m/s. The temperature of the hydrous gel (4) increased to 120°C after 10 minutes, and became 150°C after 20 minutes.

The hydrous gel particles were screened by a screener 300, and a water-absorbent resin (16) was obtained. The water-absorbent resin (16) contained a large number of bubbles apparently resulted from sudden boiling. The average particle diameter and the logarithm standard deviation $\sigma\zeta$ of this water-absorbent resin (10) were 4.0mm and 0.18, respectively. The content of residual monomer was 280ppm. The dust amount was 2520mg/m3.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 16]

The same method as that of Example 8 was performed except that, in the cutting process, propyleneglycol was sprayed at 60g/min from the respective 2-fluid nozzles (BIMV11002: product of H.Ikeuchi&Co., Ltd) of the spraying devices 9, 10 and 11 under 0.1MPa of air pressure. Further, propyleneglycol aqueous solution was sprayed from a gear pump of the spraying device 12 at 60g/min. The whole amount of propyleneglycol was 38.10 wt% with respect to the solid content of water-absorbent resin. The hydrous gel particles were screened by a screener 300, and a water-absorbent resin (17) was obtained. The average particle diameter of this water-absorbent resin (17) was 4.1mm. The content of residual monomer was 225ppm. The dust amount was 3020mg/m$^3$.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Example 17]

The same method as that of Example 13 was performed except that the monomer aqueous solution adjusted to 20 °C was supplied at 1.4kg/min from the liquid inlet of a 2-fluid nozzle, and nitrogen gas was sent to the monomer aqueous solution from the gas inlet at 200ml/min. The defoaming device (QUICK TORON® 01: Nippon Oil Corporation) was not used. The same processes after polymerization as those of Example 13 was performed with respect to this monomer aqueous solution.

The moisture content of the resulting gel was 45.3 mass %, and the thickness was 4.5 mm. The hydrous gel contained a large number of bubbles. The hydrous gel having been cut into particles contain 6-plane particles at a ratio of 90 mass%. The mass average particle diameter and the logarithm standard deviation of this hydrous gel were 4.6mm and 0.24, respectively.

The hydrous gel particles were screened by a screener 300, and a water-absorbent resin (18) was obtained. The average particle diameter and the logarithm standard deviation σζ of this water-absorbent resin (18) were 4.2mm and 0.23, respectively. Further, CRC was 42.4g/g, and the content of residual monomer was 230ppm. The dust amount was 200mg/m3. The water-absorbent resin (18) contained a large number of visible bubbles.

Note that, the measurements of mass average particle diameter and the logarithmic standard deviation of the hydrous gelatinous polymer or the water-absorbent resin were carried out by using plural sieves, which are 8.0mm, 6.70mm, 5.60mm, 4.75mm, 4.0mm, 3.35mm, 2.8mm, 2.0mm in mesh size, respectively.

[Comparative Example 1]

A hydrous gel sheet was produced by the method of Example 1, and the gel sheet was cut by scissors into 1 mm blocks so as to produce an angular hydrous gel with 6 smooth planes (5). The resulting hydrous gel grains aggregated. The amount of 6-plane particles was 100 mass %. The mass average particle diameter and the logarithm standard deviation of this hydrous gel were 1.0mm and 0.20, respectively.

The hydrous gel was spread on a 50 metal gauze (mesh size = 300 μm), and dried for 30 minutes at 130 °C. The resulting gel was a chunk in which the particles were fused into each other. The moisture content of the water-absorbent resin was 16.5 mass %, and CRC was 41.0g/g. The content of residual monomer was 450ppm. Yellowing was observed in the gel in the light resistance test.

[Comparative Example 2]

A hydrous gel sheet was produced by the method of Example 1, except that the polyethyleneglycol was excluded, and the gel sheet was cut by scissors into 4 mm blocks so as to produce an angular hydrous gel with 6 planes (6). The amount of 6-plane particles was 100 mass %. The mass average particle diameter and the logarithm standard deviation of this hydrous gel were 4.0mm and 0.20, respectively. The resulting hydrous gel grains re- aggregated.

The hydrous gel was spread on a 50 metal gauze (mesh size = 300 μm), and dried for 30 minutes at 130 °C. The resulting gel was a chunk in which the particles were fused into each other. The moisture content of the water-absorbent resin was 24.0 mass %, and CRC was 41.0g/g. The content of residual monomer was 550ppm.

The production method of water-absorbent resin according to the present invention is preferably arranged so that: in the step (5), the particles smaller or greater than the predetermined range are removed so that the water-absorbent resin is constituted of particles 2mm to 10mm in mass average particle diameter.

By removing the excessively-small particles and excessively-large particles from the water-absorbent resin adjusted to have the specific mass average particle diameter, the display effect of the water-absorbent resin further improves.

The production method of water-absorbent resin according to the present invention is preferably arranged so that: the step (2a) or (2b) is performed with a cutting device coated with at least one coating material. On this account, the defects in the cutting process due to clogging of hydrous gelatinous polymer particles can be prevented.

The production method of water-absorbent resin according to the present invention is preferably arranged so that: in the step (2a) or (2b), the hydrous gelatinous polymer is cut into hydrous gelatinous polymer particles containing angular particles, each of which has 6 smooth planes, the hydrous gelatinous polymer particles containing said angular particles in an amount of 50 mass % to 100 mass % with respect to the mass of the hydrous gelatinous polymer. The hexagonal particles are produced by simply vertically or horizontally cutting the hydrous gelatinous polymer. Therefore, cutting process can be performed more efficiently and the size of hydrous gelatinous polymer can be easily adjusted.

The production method of water-absorbent resin according to the present invention is preferably arranged so that: the step (3) includes a sub-step of splaying and/or diffusing the adhesion block agent onto a vertical-cutting blade, a horizontal-cutting spinning blade, a fixed blade and/or the hydrous gelatinous polymer. With this arrangement, the adhesion block agent is either directly adhered to the hydrous gelatinous polymer, or indirectly through the vertical and/or horizontal spinning blade, or the fixed blade, thereby coating the hydrous gelatinous polymer. The hydrous gelatinous polymer particles are therefore not adhered to each other. The re-aggregation of the hydrous gelatinous polymer particles after cutting can be thus prevented. Moreover, as this also has an effect of lubricating the particles to be released from the vertical and/or horizontal spinning blade, or the fixed blade, the cutting property of the vertical and/or horizontal spinning blade, and the fixed blade are maintained.

The production method of water-absorbent resin according to the present invention is preferably arranged so that: the adhesion block agent is sprayed and/or diffused onto the vertical-cutting blade, the horizontal-cutting spinning blade, the fixed blade and/or the hydrous gelatinous polymer so that the content of the adhesion block agent in the hydrous gelatinous polymer falls within a range of 0.0015 mass % to 35 mass % with respect to the solid content of the water-absorbent resin. With this arrangement, at least a part of the surface of each particle of the hydrous gelatinous polymer is coated. Therefore the adhesion between the particles can be more effectively prevented. Moreover, it has an effect of facilitating the drying of particles. The content of residual monomer is therefore reduced.

The production method of water-absorbent resin according to the present invention is preferably arranged so that: in the step (1), a surfactant and/or a multivalent alcohol is included into an ethylenically-unsaturated monomer aqueous solution containing acrylic acid and/or acrylate at a ratio of 30 mole% to 100 mole% (excluding a crosslinking agent), and/or into the hydrous gelatinous polymer. With this arrangement, the surfactant or the multivalent alcohol gives an effect of preventing adhesion between the particles. Therefore the adhesion preventing property by way of surface coating can be further ensured. As a result, the content of residual monomer of the water-absorbent resin can be further reduced.

Further, by carrying out a mixing process as a coating process in addition to the cutting process, it is possible to mix the polymer and the adhesion block agent by spraying/diffusing the agent into the polymer.

The production method of water-absorbent resin according to the present invention preferably further comprises the step of: (6) applying a chelate agent and/or an ultraviolet absorbent onto the surface including cross-section of the hydrous gelatinous polymer, and/or including the chelate agent and/or the ultraviolet absorbent into the hydrous gelatinous polymer. Metal ion and ultraviolet can be causes of degrading the water-absorbent resin. In the foregoing arrangement, the metal ion is trapped by a chelate agent, and the ultraviolet is absorbed by an ultraviolet absorber. The resulting water-absorbent resin has high durability, particularly for light etc.

The production method of water-absorbent resin according to the present invention is preferably arranged so that: the hydrous gelatinous polymer particles having been coated with the adhesion block agent are dried under a temperature of 80°C to 150°C so that the moisture content of the water-absorbent resin becomes 5 mass % to 30 mass %, and the content of residual monomer of the water-absorbent resin becomes 0 mass ppm to 300 mass ppm. With this arrangement, the hydrous gelatinous polymer particles may be dried well without inducing bubbles. Consequently the content of residual monomer can be reduced. The resulting water-absorbent resin is immune to odor or other sanitary problems. Moreover, the water-absorbent resin particles are not aggregate and superior in display effect.

The production method of water-absorbent resin according to the present invention preferably further comprises the step of: (7) drying the hydrous gelatinous polymer particles having been coated with the adhesion block agent under a temperature of 10°C to 120°C.

In this method, the water-absorbent resin is dried under temperature of 10°C to 120°C. Therefore the dust amount can be sufficiently reduced.

The production method of water-absorbent resin according to the present invention is preferably arranged so that: the sheet-type ethylenically-unsaturated monomer has a thickness of 3mm to 10mm, and the water-absorbent resin having been through the steps (2a) or (2b), (3) and (4) is 2mm to 10mm in mass average particle diameter, and 0 to 0.25 in logarithm standard deviation of particle size distribution.

The foregoing water-absorbent resin thus specified in mass average particle diameter and logarithm standard deviation of particle size distribution is produced by drying hydrous gelatinous polymer particles uniform in particle size. More specifically, the resin is produced by drying a hydrous gelatinous polymer in which the residual monomer and the residual polymerization initiator can be easily reacted. It is therefore possible to reduce residual monomer of the resulting water-absorbent resin.

The cutting device according to the present invention is preferably arranged so that the cutting device is coated with at least one coating material. On this account, the clogging of the hydrous gelatinous polymer in the cutting device can be suppressed.

The water-absorbent resin according to the present invention is preferably arranged so that: the water-absorbent resin is screened so that the water-absorbent resin becomes 2mm to 10mm in mass average particle diameter and 0 to 0.25 in logarithm standard deviation of particle size distribution. By subjecting the water-absorbent resin to the screening process, the resulting water-absorbent resin is superior in display effect.

The water-absorbent resin according to the present invention is preferably arranged so that: the content of residual monomer of the water-absorbent resin is 0 mass ppm to 300 mass ppm. The water-absorbent resin is produced by drying a hydrous gelatinous polymer in which the residual monomer and the residual polymerization initiator can be easily reacted. It is therefore possible to reduce residual monomer (within the foregoing range) of the resulting water-absorbent resin.

The water-absorbent resin according to the present invention is preferably arranged so that: the moisture content of the water-absorbent resin is 5 mass % to 30 mass %. The water-absorbent resin is produced by drying a hydrous gelatinous polymer whose moisture content is suitable to cause reaction between the residual monomer and the polymerization initiator. Consequently, the moisture content of the water-absorbent resin falls within 5 mass % to 30 mass % with respect to the mass of the water-absorbent resin. It is therefore possible to reduce residual monomer of the resulting water-absorbent resin.

A water-absorbent resin according to the present invention is produced by drying hydrous gelatinous polymer angular particles, at least a part of the surface of each of the hydrous gelatinous polymer particles is coated with an adhesion block agent containing at least one kind of compounds classified into multivalent metallic salt, multivalent alcohol or a surfactant.

With this arrangement, the adhesion block agent coating at least a part of each angular particle serves as a lubricant. Therefore, the hydrous gelatinous polymer particles do not easily aggregate, and therefore the adhesion between the hydrous gelatinous polymer particles is significantly reduced. Further, even when the particles aggregate, they are easily separated into individual particles again by applying a slight external force. With this property the particles can be easily dried and the residual monomer amount can be reduced.

The water-absorbent resin as set forth in claim 20 according to the present invention is preferably arranged so that: the content of the adhesion block agent falls within a range of 0.0015 mass % to 35 mass % with respect to the solid content of the water-absorbent resin. With this arrangement, the amount of adhesion block agent is adjusted to a suitable level to prevent the aggregation of the water-absorbent resin. Consequently, the resulting water-absorbent resin particles are uniform in shape. The particles can be efficiently dried and contain less amount of residual monomer.

The water-absorbent resin according to the present invention is preferably arranged so that: the water-absorbent resin contains angular particles each of which has 6 smooth planes in an amount of 50 mass % to 100 mass % with respect to the mass of the hydrous gelatinous polymer. The hexagonal particles can be easily produced by vertically and horizontally cutting the hydrous gelatinous polymer. The water-absorbent resin mainly containing the hexagonal particles includes a less amount of residual monomer. Such particles can be easily produced at low cost.

The water-absorbent resin according to the present invention is preferably arranged so that: the water-absorbent resin further contains a chelate agent and/or an ultraviolet absorbent. With this arrangement, Metal ion and ultraviolet can be causes of degrading the water-absorbent resin. In the foregoing arrangement, the metal ion is trapped by a chelate agent, and the ultraviolet is absorbed by an ultraviolet absorber. It is thus possible to improve durability of water-absorbent resin, particularly for water-absorbent resin for external use.

In the water-absorbent resin production method according to the present invention, at least a part of surface of each particle is coated with an adhesion block agent containing at least one kind of compounds classified into multivalent metal salt, multivalent alcohol, or surfactant at least partially by a specific method. The particles are completed through the subsequent drying. The method of the present invention thus produces particles which can be efficiently dried and include a less amount of residual monomer.

The present invention thus provides a method of manufacturing a water-absorbent resin by which the physicality of the resulting resin does not significantly decrease. The water-absorbent resin of the present invention is suitable for a plant-raising water retaining agent, an artificial ice for display or the like.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.


**Claims**

1. A production method of water-absorbent resin, **characterized by** comprising the steps of:

> (1) carrying out stationary polymerization of a sheet-type ethylene unsaturated monomer containing acrylic acid and/or acrylate at a ratio of 30 mole% to 100 mole% (excluding a crosslinking agent) so as to produce a hydrous gelatinous polymer containing moisture at a ratio of 40 mass % to 70 mass %;

(2a) cutting the hydrous gelatinous polymer so as to produce hydrous gelatinous polymer particles, the hydrous gelatinous polymer particles containing particles 3mm to 10mm in mass average particle diameter, each of which has 4 - 12 planes, the hydrous gelatinous polymer particles containing said particles in an amount of 50 mass % to 100 mass % with respect to the mass of the hydrous gelatinous polymer; or

(2b) drying the hydrous gelatinous polymer until its moisture content becomes 5 mass % to 60 mass % and then cutting the hydrous gelatinous polymer so as to produce hydrous gelatinous polymer particles, the hydrous gelatinous polymer particles containing particles 2mm to 10mm in mass average particle diameter, each of which has 4 - 12 planes, the hydrous gelatinous polymer particles containing said particles in an amount of 50 mass % to 100 mass % with respect to the mass of the hydrous gelatinous polymer;

(3) coating at least a part of the surface of each of the hydrous gelatinous polymer particles with an adhesion block agent containing at least one kind of compounds classified into multivalent metallic salt, multivalent alcohol or a surfactant, the coating being performed before, after, and/or at the time of cutting the hydrous gelatinous polymer; and

(4) drying the hydrous gelatinous polymer particles having been coated by adhesion block agent.

2. The production method of water-absorbent resin as set forth in claim 1, further comprising the step of:

(5) screening a water-absorbent resin resulted from the drying of the hydrous gelatinous polymer particles.

3. The production method of water-absorbent resin as set forth in claim 2, wherein:

in the step (5), the particles smaller or greater than the predetermined range are removed so that the water-absorbent resin is constituted of particles 2mm to 10mm in mass average particle diameter.

4. The production method of water-absorbent resin as set forth in any one of claims 1 through 3, wherein:

the step (2a) or (2b) is a production method of a water-absorbent resin using a cutting device, the cutting device includes a vertical-cutting blade (1, 2), a scraper (3, 4), a horizontal-cutting spinning blade (200), and a casing (14), at least one of which is coated with one of non-adhesive paint, lubricative paint, and a metallic material.

5. The production method of water-absorbent resin as set forth in any one of claims 1 through 4, wherein:

in the step (5), the hydrous gelatinous polymer is cut into hydrous gelatinous polymer particles containing angular particles, each of which has 6 smooth planes, the hydrous gelatinous polymer particles containing said angular particles in an amount of 50 mass % to 100 mass % with respect to the mass of the hydrous gelatinous polymer.

6. The production method of water-absorbent resin as set forth in any one of claims 1 through 5, wherein:

the step (3) includes a sub-step of splaying and/or diffusing the adhesion block agent onto a vertical-cutting blade (1, 2), a horizontal-cutting spinning blade (200), a fixed blade (8) and/or the hydrous gelatinous polymer.

7. The production method of water-absorbent resin as set forth in claim 6, wherein:

the adhesion block agent is sprayed and/or diffused onto the vertical-cutting blade (1, 2), the horizontal-cutting spinning blade (200), the fixed blade (8) and/or the hydrous gelatinous polymer so that the content of the adhesion block agent in the hydrous gelatinous polymer falls within a range of 0.0015 mass % to 35 mass % with respect to the solid content of the water-absorbent resin.

8. The production method of water-absorbent resin as set forth in any one of claims 1 through 7, wherein:

in the step (1), a surfactant and/or a multivalent alcohol is included into an ethylene unsaturated monomer aqueous solution containing acrylic acid and/or acrylate at a ratio of 30 mole% to 100 mole% (excluding a crosslinking agent), and/or into the hydrous gelatinous polymer.

9. The production method of water-absorbent resin as set forth in any one of claims 1 through 8, further comprising the step of:

(6) applying a chelate agent and/or an ultraviolet absorbent onto the surface including cross-section of the hydrous gelatinous polymer, and/or including the chelate agent and/or the ultraviolet absorbent into the hydrous gelatinous polymer.

**10.** The production method of water-absorbent resin as set forth in any one of claims 1 through 9, wherein:

the hydrous gelatinous polymer particles having been coated with the adhesion block agent are dried under a temperature of 80°C to 150°C so that the moisture content of the water-absorbent resin becomes 5 mass % to 30 mass %, and the content of residue monomer of the water-absorbent resin becomes 0 mass ppm to 300 mass ppm.

**11.** The production method of water-absorbent resin as set forth in any one of claims 2 through 10, further comprising the step of:

(7) drying the hydrous gelatinous polymer particles having been coated with the adhesion block agent under a temperature of 10°C to 120°C.

**12.** The production method of water-absorbent resin as set forth in any one of claims 1 through 11, wherein:

the sheet-type ethylene unsaturated monomer has a thickness of 3mm to 10mm, and the water-absorbent resin having been through the steps (2a) or (2b), (3) and (4) is 2mm to 10mm in mass average particle diameter, and 0 to 0.25 in logarithm standard deviation of particle size distribution.

**13.** A cutting device (100) for cutting a sheet-type hydrous gelatinous polymer, comprising:

at least one vertical-cutting blade (1, 2) for vertically cutting the hydrous gelatinous polymer;
at least one horizontal-cutting spinning blade (200) and at least one fixed blade (8) for vertically cutting the hydrous gelatinous polymer; and
at least one spraying means (9, 10, 11, 12) for spraying and/or diffusing an adhesion block agent onto the vertical-cutting blade, the horizontal-cutting spinning blade, the fixed blade and/or the hydrous gelatinous polymer.

**14.** The cutting device as set forth in claim 13 wherein the cutting device (100) includes a vertical-cutting blade (1, 2), a scraper (3, 4), a horizontal-cutting spinning blade (200), and a casing (14), at least one of which is coated with one of non-adhesive paint, lubricative paint, and a metallic material.

**15.** A water-absorbent resin, **characterized by** being produced through the steps of:

(1) carrying out stationary polymerization of an ethylene unsaturated monomer containing acrylic acid and/or acrylate at a ratio of 30 mole% to 100 mole% so as to produce a hydrous gelatinous polymer;
(2) cutting the hydrous gelatinous polymer so as to produce hydrous gelatinous polymer particles containing particles each of which has 4 - 12 planes, the hydrous gelatinous polymer particles containing said particles in an amount of 50 mass % to 100 mass % with respect to the mass of the hydrous gelatinous polymer; and
(3) drying the hydrous gelatinous polymer particles, the water-absorbent resin being 2mm to 10mm in mass average particle diameter, and 0 to 0.25 in logarithm standard deviation of particle size distribution.

**16.** The water-absorbent resin as set forth in claim 15, wherein:

the water-absorbent resin is screened so that the water-absorbent resin becomes 2mm to 10mm in mass average particle diameter and 0 to 0.25 in logarithm standard deviation of particle size distribution.

**17.** The water-absorbent resin as set forth in claim 15 or 16, wherein:

the content of residue monomer of the water-absorbent resin is 0 mass ppm to 300 mass ppm.

**18.** The water-absorbent resin as set forth in any one of claims 15 through 17, wherein:

the moisture content of the water-absorbent resin is 5 mass % to 30 mass %.

**19.** A water-absorbent resin, **characterized by** being produced by drying hydrous gelatinous polymer angular particles, at least a part of the surface of each of the hydrous gelatinous polymer particles is coated with an adhesion block agent containing at least one kind of compounds classified into multivalent metallic salt, multivalent alcohol or a surfactant.

**20.** The water-absorbent resin as set forth in claim 19, wherein:

the content of the adhesion block agent falls within a range of 0.0015 mass % to 35 mass % with respect to the solid content of the water-absorbent resin.

**21.** The water-absorbent resin as set forth in any one of claims 15 through 20, wherein:

the water-absorbent resin contains angular particles each of which has 6 smooth planes in an amount of 50 mass % to 100 mass % with respect to the mass of the hydrous gelatinous polymer.

**22.** The water-absorbent resin as set forth in any one of claims 15 through 21, wherein:

the water-absorbent resin further contains a chelate agent and/or an ultraviolet absorbent.

**23.** A screener (300) for screening a water-absorbent resin comprising a gas spraying mechanism (20).

**24.** A plant-raising water retaining agent comprising (i) a water-absorbent resin produced through a method as set forth in any one of claims 1 through 12, and/or (ii) a water-absorbent resin as set forth in any one of claims 15 through 22.

**25.** An artificial ice for display comprising (i) a water-absorbent resin produced through a method as set forth in any one of claims 1 through 12, and/or (ii) a water-absorbent resin as set forth in any one of claims 15 through 22.

FIG. 1

FIG. 2

**FIG. 3**

**FIG. 4**

**FIG. 5**

**FIG. 6**

**FIG. 7**

FIG. 8

FIG. 9

FIG. 10

FIG. 11

## FIG. 12

## FIG. 13

600

FIG. 14

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8092307 A
- JP 61110510 A
- JP 11349687 A
- US 4973632 A
- US 5998553 A
- JP 63007203 A
- US 20040068057 A
- JP 7098847 A
- JP 4106108 A
- US 5229488 A
- JP 5040780 A